# EUROPEAN PATENT SPECIFICATION

(11) **EP 2 074 429 B1**
(45) Date of publication and mention of the grant of the patent: **04.04.2012**
(21) Application number: 07818669.9
(22) Date of filing: 04.10.2007
(51) Int. Cl.: G01N 33/68, C12Q 1/68

(54) **CTGF AS A BIOMARKER, THERAPEUTIC AND DIAGNOSTIC TARGET**
CTGF ALS BIOMARKER, THERAPEUTISCHES UND DIAGNOSTISCHES ZIEL
CTGF UTILISÉ COMME BIOMARQUEUR, CIBLE THÉRAPEUTIQUE ET DIAGNOSTIQUE

(30) Priority: 16.10.2006 EP 06021596
(43) Date of publication of application: 01.07.2009
(73) Proprietor: Bayer Pharma Aktiengesellschaft, 13353 Berlin (DE)
(72) Inventor: GOLZ, Stefan, 45326 Essen (DE); SUMMER, Holger, 42113 Wuppertal (DE); GEERTS, Andreas, 42113 Wuppertal (DE); BRÜGGEMEIER, Ulf, 42799 Leichlingen (DE); ALBRECHT-KÜPPER, Barbara, 42289 Wülfrath (DE); KLEIN, Martina, 40489 Düsseldorf (DE); STEPPAN, Sonja, 63263 Neu-Isenburg (DE); ELLINGHAUS, Peter, 49324 Melle (DE); D'URSO, Donatella, 40547 Duesseldorf (DE); SEEWALD, Michael, 10179 Berlin (DE); MILTING, Hendrik, 32545 Bad Oeynhausen (DE)
(86) International application number: PCT/EP2007/008590
(87) International publication number: WO 2008/046517

(56) References cited:
- WO-A-03/024308
- WO-A-2005/070446
- US-A1- 2005 136 502
- VALLON VOLKER ET AL: "SGK1-dependent cardiac CTGF formation and fibrosis following DOCA treatment" JOURNAL OF MOLECULAR MEDICINE (BERLIN), vol. 84, no. 5, May 2006 (2006-05), pages 396-404, XP002464350 ISSN: 0946-2716(print) 1432-1440(ele
- OHNISHI HIROMICHI ET AL: "Increased expression of connective tissue growth factor in the infarct zone of experimentally induced myocardial infarction in rats" JOURNAL OF MOLECULAR AND CELLULAR CARDIOLOGY, vol. 30, no. 11, November 1998 (1998-11), pages 2411-2422, XP002464351 ISSN: 0022-2828
- LEE YOUNG-SAM ET AL: "Monocrotaline-induced pulmonary hypertension correlates with upregulation of connective tissue growth factor expression in the lung" EXPERIMENTAL & MOLECULAR MEDICINE, vol. 37, no. 1, February 2005 (2005-02), pages 27-35, XP002464352 ISSN: 1226-3613
- RODRIGUE-WAY A ET AL: "Sarcomeric genes involved in reverse remodeling of the heart during left ventricular assist device support" JOURNAL OF HEART AND LUNG TRANSPLANTATION, MOSBY-YEAR BOOK, INC., ST LOUIS, MO, US, vol. 24, no. 1, January 2005 (2005-01), pages 73-80, XP004711594 ISSN: 1053-2498
- CAMPIAN MARIA E ET AL: "How valid are animal models to evaluate treatments for pulmonary hypertension?", NAUNYN-SCHMIEDEBERG'S ARCHIVES OF PHARMACOLOGY, vol. 373, no. 6, September 2006 (2006-09), pages 391-400, ISSN: 0028-1298

## Description

### Technical field of the invention

The present invention is in the field of molecular biology, more particularly, the present invention relates to nucleic acid sequences and amino acid sequences of a human and rat CTGF and its regulation for the treatment, diagnostic and use as a biomarker of cardiovascular diseases and hematological diseases in mammals.

### Background of the invention

### TaqMan-Technology / expression profiling

TaqMan is a recently developed technique, in which the release of a fluorescent reporter dye from a hybridisation probe in real-time during a polymerase chain reaction (PCR) is proportional to the accumulation of the PCR product. Quantification is based on the early, linear part of the reaction, and by determining the threshold cycle (CT), at which fluorescence above background is first detected.

Gene expression technologies may be useful in several areas of drug discovery and development, such as target identification, lead optimization, and identification of mechanisms of action. The TaqMan technology can be used to compare differences between expression profiles of normal tissue and diseased tissue. Expression profiling has been used in identifying genes, which are up- or downregulated in a variety of diseases. An interesting application of expression profiling is temporal monitoring of changes in gene expression during disease progression and drug treatment or in patients versus healthy individuals. The premise in this approach is that changes in pattern of gene expression in response to physiological or environmental stimuli (e.g., drugs) may serve as indirect clues about disease-causing genes or drug targets. Moreover, the effects of drugs with established efficacy on global gene expression patterns may provide a guidepost, or a genetic signature, against which a new drug candidate can be compared.

### CTGF

The nucleotide sequence of CTGF is accessible in the databases by the accession number M92934 (human) and AF120275 (rat). The sequences are given in SEQ ID NO:1 (human) and SEQ ID NO:2 (rat). The amino acid sequence of CTGF depicted in SEQ ID NO:3 (human) and SEQ ID NO:4 (rat).

Connective tissue growth factor (CTGF) also known as CCN2, is a 38-kDa multifunctional growth factor that is a member of the CCN [CYR61 (cysteine-rich 61) / CTGF / NOV (nephroblastoma overexpressed)] family of secreted proteins, which are characterized as cysteine-rich matricellular proteins that each contain four modular domains displaying homology to insulin-like growth factor-binding proteins (domain 1), a von Willebrand factor type C repeat (domain 2), a thrombospondin type 1 repeat (domain 3), and a cysteine knot domain (domain 4), respectively. [Bork et al (1993)]. The cysteine knot domain contains heparin-binding sites that mediate binding to extracellular matrix and cell surface heparan sulfate proteoglycans [Chen et al., (2001)]. CTGF is an immediate early gene that is potently induced by a variety of stimuli that regulate extracellular matrix deposition, tissue remodeling, and neovascularization, including platelet-derived growth factor, transforming growth factor (TGF)-h, basic fibroblast growth factor, vascular endothelial growth factor (VEGF), and hypoxia in fibroblasts or endothelial cells [Igarashi et al., (1993); Grotendorst et al. (1996); Shima et al. (2001); Suzuma ct al., (2000)]. CTGF exhibits a diverse range of cellular functions including cell adhesion, stimulation of cell migration, and potentiation of growth factor-induced DNA synthesis [Takigawa et al. (2000)]. CTGF interacts with integrin receptors including avh3, aIIbh3, a6h1, and amh2 [Chen et al., (2001); Gao et al. (2004); Jedsadayanmata et al., (1999); Schober et al., (2002)] and has been reported to be a ligand for low-density lipoprotein-related protein 1 (LRP-1); interacts with LRP-5 to inhibit Wnt signaling [Gao et al., (2003)] and can interact directly with several growth factors including TGF-h [Roestenberg et al. (2004)]. Taken together, past studies indicate that the mechanism of action of CTGF relates to its capacity to modulate and amplify a variety of biological processes by binding directly to mitogenic, fibrogenic, and angiogenic factors that are important in inflammation and fibrosis.

CTGF is published (but not limited to) in patents WO2004075835 and WO02068579.

Vallon Volker et al describe SGK1-dependent cardiac CTGF formation and fibrosis following DOCA treatment (JOURNAL OF MOLECULAR MEDICINE, vol. 84, no. 5, 2006 pages 396-404). Ohnishi Hiromichi et al describe the increased expression of connective tissue growth factor in the infarct zone of experimentally induced myocardial infarction in rats (JOURNAL OF MOLECULAR AND CELLULAR CAROIOLOGY, vol. 30, no. 11, 1998, page 2411). Further, Lee Young-Sam et al disclose that monocrotaline-induced pulmonary hypertension correlates with upregulation of connective tissue growth factor expression in the lung (EXPERIMENTAL & MOLECULAR MEDICINE, vol. 37, no. 1, 2005, page 27). Rodrigue-Way et al describe sarcomeric genes involved in reverse remodeling of the heart du ring left ventricular assist device support (JOURNAL OF HEART ANO LUNG TRANSPLANTATION, MOSBY-YEAR BOOK, INC., ST LOUIS, MO, US, vol. 24, no. 1, 2005, pages 73-80). WO03/024308, WO2005/070446 and US2005136502 disclose antibodies, primes and probes detecting CTGF protein or polynucleotide.

### Summary of The invention

The invention relates to the use CTGF as a disease, efficacy or surrogate end point biomarker for a therapy of pulmonary hypertension.

### Brief Description of the Drawings

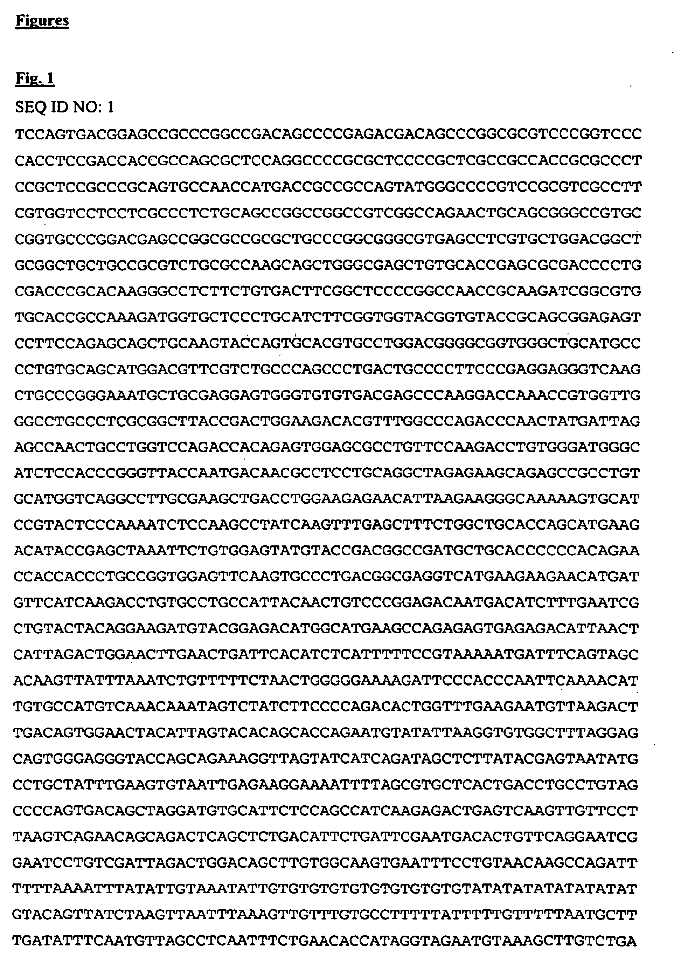

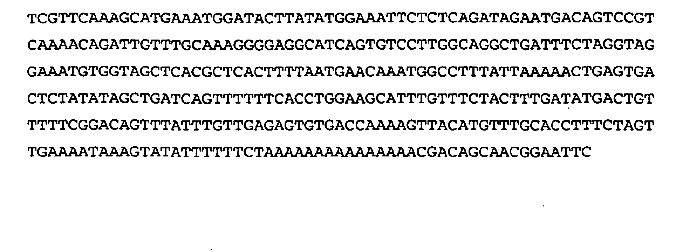

- Fig. 1: shows the nucleotide sequence of a CTGF polynucleotide human (SEQ ID NO: 1).

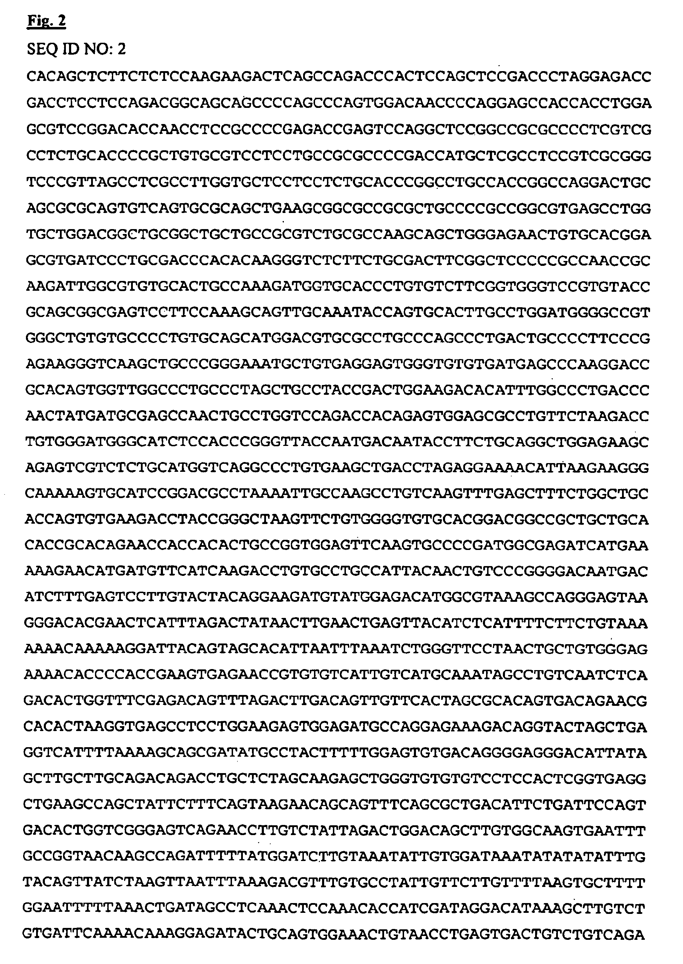

- Fig. 2: shows the nucleotide sequence of a CTGF polynucleotide rat (SEQ ID NO:2).

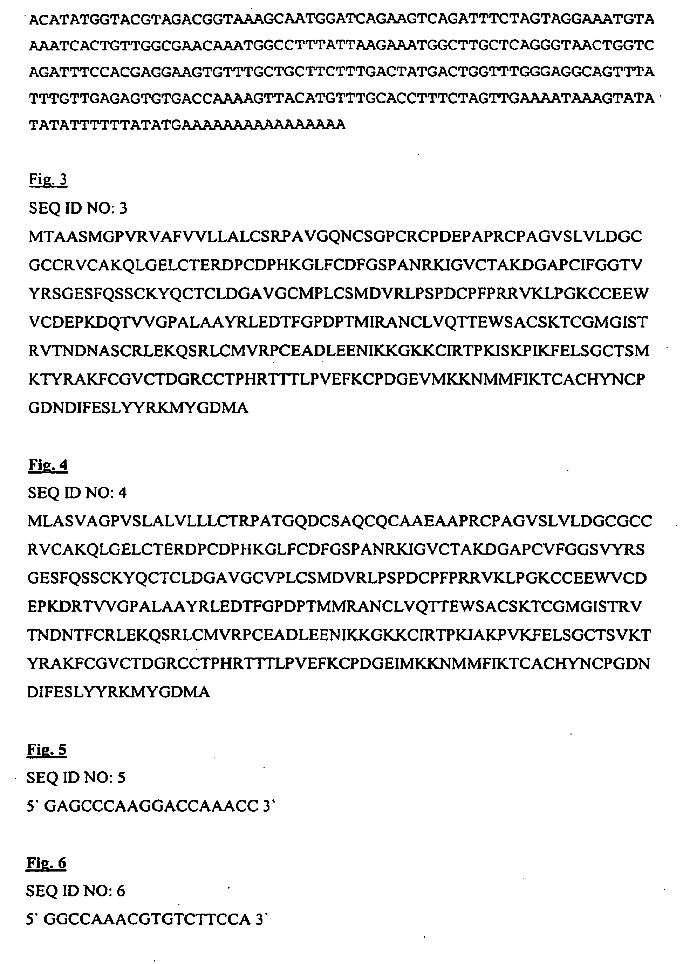

- Fig. 3: shows the amino acid sequence of a CTGF polypeptide human (SEQ ID NO:3).
- Fig. 4: shows the amino acid sequence of a CTGF polypeptide rat (SEQ ID NO:4).
- Fig. 5: shows the nucleotide sequence of a primer useful for the invention (SEQ ID NO: 5).
- Fig. 6: shows the nucleotide sequence of a primer useful for the invention (SEQ ID NO:6).
- Fig. 7: shows a nucleotide sequence useful as a probe to detect proteins of the invention (SEQ ID NO:7).
- Fig. 8: shows the nucleotide sequence of a primer useful for the invention (SEQ ID NO:8).
- Fig. 9: shows the nucleotide sequence of a primer useful for the invention (SEQ ID NO:9).
- Fig. 10: shows a nucleotide sequence useful as a probe to detect proteins of the invention (SEQ ID NO:10).
- Fig. 11: shows the results of real-time expression analysis of CTGF in rat hearts (DOCA). X axis: treatment; Y axis : relative expression; A : control; B: control/placebo; C1 : compound P / concentration 1; C2 : compound P / concentration 2; C3 : compound P / concentration. The expression of CTGF is disease state, treatment- and dose-dependent regulated in the animal model.
- Fig. 12: shows the results of real-time expression analysis of CTGF in rat hearts (occlusion). X axis: treatment; Y axis : relative expression; A : control; B: control/placebo; C1 : compound P / concentration 1; C2 : compound P / concentration 2; C3 : compound P / concentration. The expression of CTGF is disease state, treatment- and dose-dependent regulated in the animal model.
- Fig. 13: shows the results of real-time expression analysis of CTGF in rat hearts (monocrotalin). X axis : treatment; Y axis : relative expression; A : control; B: control/placebo; C1 : compound P / concentration 1; C2 : compound P / concentration 2. The expression of CTGF is disease state, treatment- and dose-dependent regulated in the animal model.

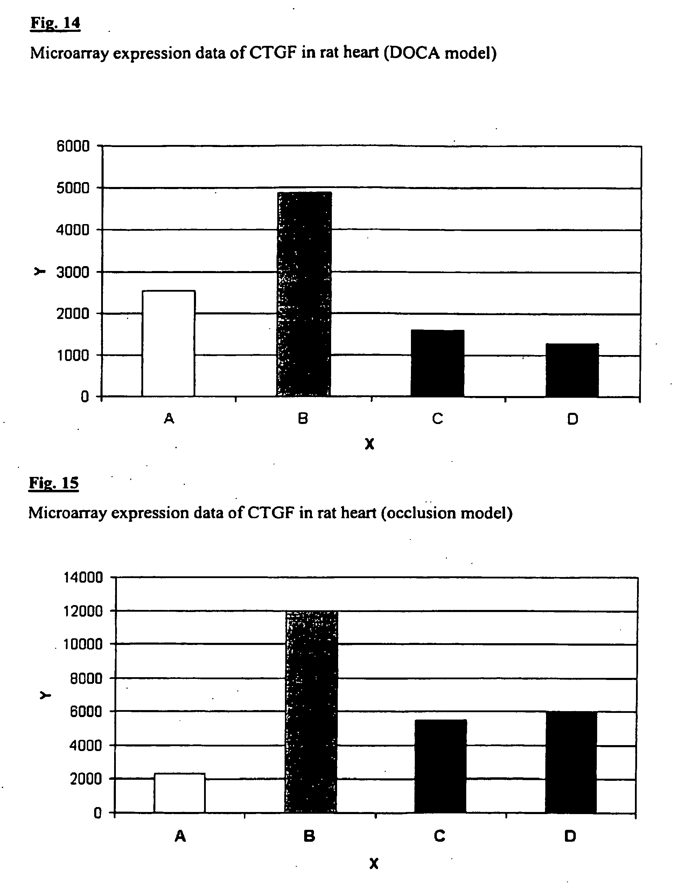

- Fig. 14: shows the results of microarray expression analysis of CTGF in rat hearts (Doca). X axis : treatment; Y axis : relative expression; A : control; B: control/placebo; C : compound P / concentration 1; D : compound P / concentration 2. The expression of CTGF is disease state, treatment- and dose-dependent regulated in the animal model.
- Fig. 15: shows the results of microarray expression analysis of CTGF in rat hearts (occlusion). X axis : treatment; Y axis : relative expression; A : control; B: control/placebo; C : compound P / concentration 1; D : compound P / concentration 2. The expression of CTGF is disease state, treatment- and dose-dependent regulated in the animal model.

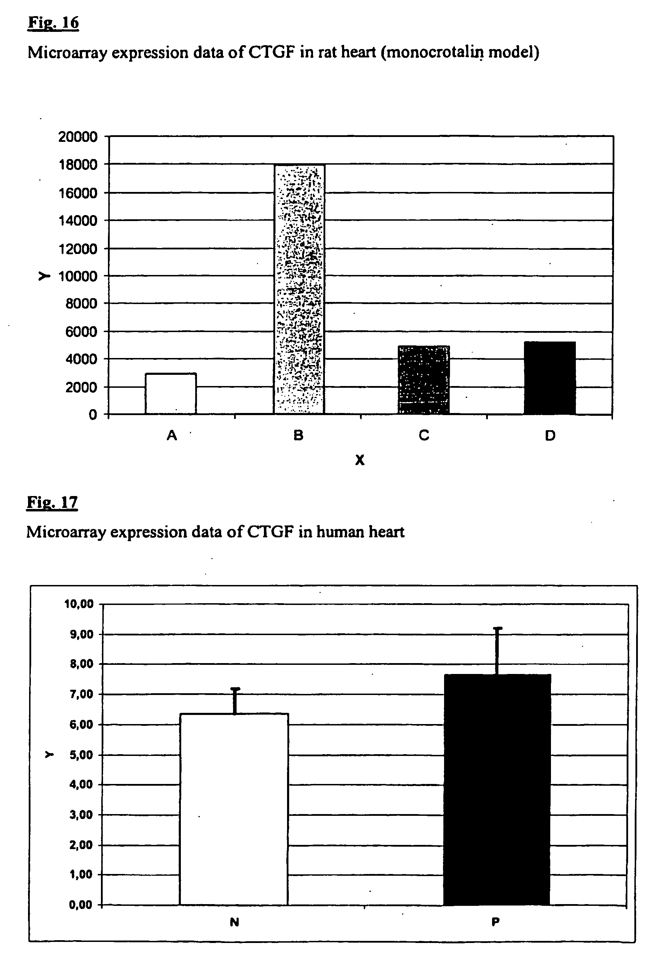

- Fig. 16: shows the results of microarray expression analysis of CTGF in rat hearts (monocrotalin). X axis : treatment; Y axis : relative expression; A : control; B: control/placebo; C : compound P / concentration 1; D : compound P / concentration 2. The expression of CTGF is disease state, treatment- and dose-dependent regulated in the animal model.
- Fig. 17: shows the results of microarray expression analysis of CTGF in human hearts. X axis : disease status; Y axis : relative expression; N : non-failure; P: pre-LVAD. The expresion of CTGF is disease state regulated in human heart.

### Detailed description of the invention

### Definition of terms

An "oligonucleotide" is a stretch of nucleotide residues which has a sufficient number of bases to be used as an oligomer, amplimer or probe in a polymerase chain reaction (PCR). Oligonucleotides are prepared from genomic or cDNA sequence and are used to amplify, reveal, or confirm the presence of a similar DNA or RNA in a particular cell or tissue. Oligonucleotides or oligomers comprise portions of a DNA sequence having at least about 10 nucleotides and as many as about 35 nucleotides, preferably about 25 nucleotides.

"Probes" may be derived from naturally occurring or recombinant single- or double-stranded nucleic acids or may be chemically synthesized. They are useful in detecting the presence of identical or similar sequences. Such probes may be labeled with reporter molecules using nick translation, Klenow fill-in reaction, PCR or other methods well known in the art. Nucleic acid probes may be used in southern, northern or in situ hybridizations to determine whether DNA or RNA encoding a certain protein is present in a cell type, tissue, or organ.

A "fragment of a polynucleotide" is a nucleic acid that comprises all or any part of a given nucleotide molecule, the fragment having fewer nucleotides than about 6 kb, preferably fewer than about 1 kb.

"Reporter molecules" are radionuclides, enzymes, fluorescent, chemiluminescent, or chromogenic agents which associate with a particular nucleotide or amino acid sequence, thereby establishing the presence of a certain sequence, or allowing for the quantification of a certain sequence.

"Chimeric" molecules may be constructed by introducing all or part of the nucleotide sequence of this invention into a vector containing additional nucleic acid sequence which might be expected to change any one or several of the following CTGF characteristics: cellular location, distribution, ligand-binding affinities, interchain affinities, degradation/turnover rate, signaling, etc.

"Active", with respect to a CTGF polypeptide, refers to those forms, fragments, or domains of a CTGF polypeptide which retain the biological and/or antigenic activity of a CTGF polypeptide.

"Naturally occurring CTGF polypeptide" refers to a polypeptide produced by cells which have not been genetically engineered and specifically contemplates various polypeptides arising from post-translational modifications of the polypeptide including but not limited to acetylation, carboxylation, glycosylation, phosphorylation, lipidation and acylation.

"Derivative" refers to polypeptides which have been chemically modified by techniques such as ubiquitination, labeling (see above), pegylation (derivatization with polyethylene glycol), and chemical insertion or substitution of amino acids such as ornithine which do not normally occur in human proteins.

"Conservative amino acid substitutions" result from replacing one amino acid with another having similar structural and/or chemical properties, such as the replacement of a leucine with an isoleucine or valine, an aspartate with a glutamate, or a threonine with a serine.

"Insertions" or "deletions" are typically in the range of about 1 to 5 amino acids. The variation allowed may be experimentally determined by producing the peptide synthetically while systematically making insertions, deletions, or substitutions of nucleotides in the sequence using recombinant DNA techniques.

A "signal sequence" or "leader sequence" can be used, when desired, to direct the polypeptide through a membrane of a cell. Such a sequence may be naturally present on the polypeptides of the present invention or provided from heterologous sources by recombinant DNA techniques.

An "oligopeptide" is a short stretch of amino acid residues and may be expressed from an oligonucleotide. Oligopeptides comprise a stretch of amino acid residues of at least 3, 5, 10 amino acids and at most 10, 15, 25 amino acids, typically of at least 9 to 13 amino acids, and of sufficient length to display biological and/or antigenic activity.

"Inhibitor" is any substance which retards or prevents a chemical or physiological reaction or response. Common inhibitors include but are not limited to antisense molecules, antibodies, and antagonists.

"Biomarker" are measurable and quantifiable biological parameters (e.g. specific enzyme concentration, specific hormone concentration, specific gene phenotype distribution in a population, presence of biological substances) which serve as indices for health - and physiology related assessments, such as disease risk, psychiatric disorders, environmental exposure and its effects, disease diagnosis, metabolic processes, substance abuse, pregnancy, cell line development, epidemiologic studies, etc.. Parameter that can be used to identify a toxic effect in an individual organism and can be used in extrapolation between species. Indicator signalling an event or condition in a biological system or sample and giving a measure of exposure, effect, or susceptibility.

Biological markers can reflect a variety of disease characteristics, including the level of exposure to an environmental or genetic trigger, an element of the disease process itself, an intermediate stage between exposure and disease onset, or an independent factor associated with the disease state but not causative of pathogenesis. Depending on the specific characteristic, biomarkers can be used to identify the risk of developing an illness (antecedent biomarkers), aid in identifying disease (diagnostic biomarkers), or predict future disease course, including response to therapy (prognostic biomarkers).

"Standard expression" is a quantitative or qualitative measurement for comparison. It is based on a statistically appropriate number of normal samples and is created to use as a basis of comparison when performing diagnostic assays, running clinical trials, or following patient treatment profiles.

"Animal" as used herein may be defined to include human, domestic (e.g., cats, dogs, etc.), agricultural (e.g., cows, horses, sheep, etc.) or test species (e.g., mouse, rat, rabbit, etc.).

A "CTGF polynucleotide", within the meaning of the invention, shall be understood as being a nucleic acid molecule selected from a group consisting of
(i) nucleic acid molecules encoding a polypeptide comprising the amino acid sequence of SEQ ID NO: 3 or SEQ ID NO: 4,
(ii) nucleic acid molecules comprising the sequence of SEQ ID NO: 1 or SEQ ID NO: 2,
(iii) nucleic acid molecules having the sequence of SEQ ID NO: 1 or SEQ ID NO: 2 ,
(iv) nucleic acid molecules the complementary strand of which hybridizes under stringent conditions to a nucleic acid molecule of (i); (ii), or (iii),
(v) nucleic acid molecules the sequence of which differs from the sequence of a nucleic acid molecule of (iii) due to the degeneracy of the genetic code,
(vi) nucleic acid molecules which have a sequence identity of at least 80%, 85%, 90%, 95%, 98% or 99%; and
(vii) wherein the polypeptide encoded by said nucleic acid molecules of (i)-(vi) have CTGF activity

A "CTGF polypeptide", within the meaning of the invention, shall be understood as being a polypeptide selected from a group consisting of
(i) polypeptides having the sequence of SEQ ID NO: 3 or 4,
(ii) polypeptides comprising the sequence of SEQ ID NO: 3 or 4,
(iii) polypeptides encoded by CTGF polynucleotides; and
(iv) polypeptides which show at least 99%, 98%, 95%, 90%, or 80% identity with a polypeptide of (i), (ii), or (iii);
wherein said polypeptide has CTGF activity.

The nucleotide sequences encoding a CTGF (or their complement) have numerous applications in techniques known to those skilled in the art of molecular biology. These techniques include use as hybridization probes, use in the construction of oligomers for PCR, use for chromosome and gene mapping, use in the recombinant production of CTGF, and use in generation of antisense DNA or RNA, their chemical analogs and the like. Uses of nucleotides encoding a CTGF disclosed herein are exemplary of known techniques and are not intended to limit their use in any technique known to a person of ordinary skill in the art. Furthermore, the nucleotide sequences disclosed herein may be used in molecular biology techniques that have not yet been developed, provided the new techniques rely on properties of nucleotide sequences that are currently known, e.g., the triplet genetic code, specific base pair interactions, etc.

It will be appreciated by those skilled in the art that as a result of the degeneracy of the genetic code, a multitude of CTGF - encoding nucleotide sequences may be produced. Some of these will only bear minimal homology to the nucleotide sequence of the known and naturally occurring CTGF. The invention has specifically contemplated each and every possible variation of nucleotide sequence that could be made by selecting combinations based on possible codon choices. These combinations are made in accordance with the standard triplet genetic code as applied to the nucleotide sequence of naturally occurring CTGF, and all such variations are to be considered as being specifically disclosed.

Although the nucleotide sequences which encode a CTGF, its derivatives or its variants are preferably capable of hybridizing to the nucleotide sequence of the naturally occurring CTGF polynucleotide under stringent conditions, it may be advantageous to produce nucleotide sequences encoding CTGF polypeptides or its derivatives possessing a substantially different codon usage. Codons can be selected to increase the rate at which expression of the peptide occurs in a particular prokaryotic or eukaryotic expression host in accordance with the frequency with which particular codons are utilized by the host. Other reasons for substantially altering the nucleotide sequence encoding a CTGF polypeptide and/or its derivatives without altering the encoded amino acid sequence include the production of RNA transcripts having more desirable properties, such as a greater half-life, than transcripts produced from the naturally occurring sequence.

Nucleotide sequences encoding a CTGF polypeptide may be joined to a variety of other nucleotide sequences by means of well established recombinant DNA techniques. Useful nucleotide sequences for joining to CTGF polynucleotides include an assortment of cloning vectors such as plasmids, cosmids, lambda phage derivatives, phagemids, and the like. Vectors of interest include expression vectors, replication vectors, probe generation vectors, sequencing vectors, etc. In general, vectors of interest may contain an origin of replication functional in at least one organism, convenient restriction endonuclease sensitive sites, and selectable markers for one or more host cell systems.

Another aspect of the present disclosure is to provide for CTGF-specific hybridization probes capable of hybridizing with naturally occurring nucleotide sequences encoding CTGF. Such probes may also be used for the detection of similar protein encoding sequences and should preferably show at least 40% nucleotide identity to CTGF polynucleotides. The hybridization probes of the subject invention may be derived from the nucleotide sequence presented as SEQ ID NO: 1 or from genomic sequences including promoter, enhancers or introns of the native gene. Hybridization probes may be labelled by a variety of reporter molecules using techniques well known in the art.

It will be recognized that many deletional or mutational analogs of CTGF polynucleotides will be effective hybridization probes for CTGF polynucleotides. Accordingly, the invention relates to nucleic acid sequences that hybridize with such CTGF encoding nucleic acid sequences under stringent conditions.

"Stringent conditions" refers to conditions that allow for the hybridization of substantially related nucleic acid sequences. For instance, such conditions will generally allow hybridization of sequence with at least about 85% sequence identity, preferably with at least about 90% sequence identity, more preferably with at least about 95% sequence identity. Hybridization conditions and probes can be adjusted in well-characterized ways to achieve selective hybridization of human-derived probes. Stringent conditions, within the meaning of the invention are 68°C in a buffer containing 0,2 x SSC (1x standard saline-citrate = 150 mM NaCl, 15 mM Trinatriumcitrat) [Sambrook et al., (1989)].

Nucleic acid molecules that will hybridize to CTGF polynucleotides under stringent conditions can be identified functionally. Without limitation, examples of the uses for hybridization probes include: histochemical uses such as identifying tissues that express CTGF; measuring mRNA levels, for instance to identify a sample's tissue type or to identify cells that express abnormal levels of CTGF; and detecting polymorphisms of CTGF.

PCR provides additional uses for oligonucleotides based upon the nucleotide sequence which encodes CTGF. Such probes used in PCR may be of recombinant origin, chemically synthesized, or a mixture of both. Oligomers may comprise discrete nucleotide sequences employed under optimized conditions for identification of CTGF in specific tissues or diagnostic use. The same two oligomers, a nested set of oligomers, or even a degenerate pool of oligomers may be employed under less stringent conditions for identification of closely related DNAs or RNAs.

Rules for designing polymerase chain reaction (PCR) primers are now established, as reviewed by PCR Protocols. Degenerate primers, i.e., preparations of primers that are heterogeneous at given sequence locations, can be designed to amplify nucleic acid sequences that are highly homologous to, but not identical with CTGF. Strategies are now available that allow for only one of the primers to be required to specifically hybridize with a known sequence. For example, appropriate nucleic acid primers can be ligated to the nucleic acid sought to be amplified to provide the hybridization partner for one of the primers. In this way, only one of the primers need be based on the sequence of the nucleic acid sought to be amplified.

PCR methods for amplifying nucleic acid will utilize at least two primers. One of these primers will be capable of hybridizing to a first strand of the nucleic acid to be amplified and of priming enzyme-driven nucleic acid synthesis in a first direction. The other will be capable of hybridizing the reciprocal sequence of the first strand (if the sequence to be amplified is single stranded, this sequence will initially be hypothetical, but will be synthesized in the first amplification cycle) and of priming nucleic acid synthesis from that strand in the direction opposite the first direction and towards the site of hybridization for the first primer. Conditions for conducting such amplification, particularly under preferred stringent hybridization conditions, are well known.

Other means of producing specific hybridization probes for CTGF include the cloning of nucleic acid sequences encoding CTGF or CTGF derivatives into vectors for the production of mRNA probes. Such vectors are known in the art, are commercially available and may be used to synthesize RNA probes in vitro by means of the addition of the appropriate RNA polymerase as T7 or SP6 RNA polymerase and the appropriate reporter molecules.

It is possible to produce a DNA sequence, or portions thereof, entirely by synthetic chemistry. After synthesis, the nucleic acid sequence can be inserted into any of the many available DNA vectors and their respective host cells using techniques which are well known in the art. Moreover, synthetic chemistry may be used to introduce mutations into the nucleotide sequence. Alternately, a portion of sequence in which a mutation is desired can be synthesized and recombined with longer portion of an existing genomic or recombinant sequence.

CTGF polynucleotides may be used to produce a purified oligo-or polypeptide using well known methods of recombinant DNA technology. The oligopeptide may be expressed in a variety of host cells, either prokaryotic or eukaryotic. Host cells may be from the same species from which the nucleotide sequence was derived or from a different species. Advantages of producing an oligonucleotide by recombinant DNA technology include obtaining adequate amounts of the protein for purification and the availability of simplified purification procedures.

### Quantitative determinations of nucleic acids

An important step in the molecular genetic analysis of human disease is often the enumeration of the copy number of a nucleis acid or the relative expression of a gene in particular tissues.

Several different approaches are currently available to make quantitative determinations of nucleic acids. Chromosome-based techniques, such as comparative genomic hybridization (CGH) and fluorescent in situ hybridization (FISH) facilitate efforts to cytogenetically localize genomic regions that are altered in tumor cells. Regions of genomic alteration can be narrowed further using loss of heterozygosity analysis (LOH), in which disease DNA is analyzed and compared with normal DNA for the loss of a heterozygous polymorphic marker. The first experiments used restriction fragment length polymorphisms (RFLPs) [Johnson, (1989)], or hypervariable minisatellite DNA [Barnes, 2000]. In recent years LOH has been performed primarily using PCR amplification of microsatellite markers and electrophoresis of the radio labelled [Jeffreys, (1985)] or fluorescently labelled PCR products [Weber, (1990)] and compared between paired normal and disease DNAs.

A number of other methods have also been developed to quantify nucleic acids [Gergen, (1992)]. More recently, PCR and RT-PCR methods have been developed which are capable of measuring the amount of a nucleic acid in a sample. One approach, for example, measures PCR product quantity in the log phase of the reaction before the formation of reaction products plateaus [Thomas, (1980)].

A gene sequence contained in all samples at relatively constant quantity is typically utilized for sample amplification efficiency normalization. This approach, however, suffers from several drawbacks. The method requires that each sample has equal input amounts of the nucleic acid and that the amplification efficiency between samples is identical until the time of analysis. Furthermore, it is difficult using the conventional methods of PCR quantitation such as gel electrophoresis or plate capture hybridization to determine that all samples are in fact analyzed during the log phase of the reaction as required by the method.

Another method called quantitative competitive (QC)-PCR, as the name implies, relies on the inclusion of an internal control competitor in each reaction [Piatak, (1993), BioTechniques]. The efficiency of each reaction is normalized to the internal competitor. A known amount of internal competitor is typically added to each sample. The unknown target PCR product is compared with the known competitor PCR product to obtain relative quantitation. A difficulty with this general approach lies in developing an internal control that amplifies with the same efficiency than the target molecule.

### 5' Fluorogenic Nuclease Assays

Fluorogenic nuclease assays are a real time quantitation method that uses a probe to monitor formation of amplification product. The basis for this method of monitoring the formation of amplification product is to measure continuously PCR product accumulation using a dual-labelled fluorogenic oligonucleotide probe, an approach frequently referred to in the literature simply as the "TaqMan method" [Piatak,(1993), Science; Heid, (1996); Gibson, (1996); Holland. (1991)].

The probe used in such assays is typically a short (about 20-25 bases) oligonucleotide that is labeled with two different fluorescent dyes. The 5' terminus of the probe is attached to a reporter dye and the 3' terminus is attached to a quenching dye, although the dyes could be attached at other locations on the probe as well. The probe is designed to have at least substantial sequence complementarity with the probe binding site. Upstream and downstream PCR primers which bind to flanking regions of the locus are added to the reaction mixture. When the probe is intact, energy transfer between the two fluorophors occurs and the quencher quenches emission from the reporter. During the extension phase of PCR, the probe is cleaved by the 5' nuclease activity of a nucleic acid polymerase such as Taq polymerase, thereby releasing the reporter from the oligonucleotide-quencher and resulting in an increase of reporter emission intensity which can be measured by an appropriate detector.

One detector which is specifically adapted for measuring fluorescence emissions such as those created during a fluorogenic assay is the ABI 7700 or 4700 HT manufactured by Applied Biosystems, Inc. in Foster City, Calif. The ABI 7700 uses fiber optics connected with each well in a 96-or 384 well PCR tube arrangement. The instrument includes a laser for exciting the labels and is capable of measuring the fluorescence spectra intensity from each tube with continuous monitoring during PCR amplification. Each tube is re-examined every 8.5 seconds.

Computer software provided with the instrument is capable of recording the fluorescence intensity of reporter and quencher over the course of the amplification. The recorded values will then be used to calculate the increase in normalized reporter emission intensity on a continuous basis. The increase in emission intensity is plotted versus time, i.e., the number of amplification cycles, to produce a continuous measure of amplification. To quantify the locus in each amplification reaction, the amplification plot is examined at a point during the log phase of product accumulation. This is accomplished by assigning a fluorescence threshold intensity above background and determining the point at which each amplification plot crosses the threshold (defined as the threshold cycle number or Ct). Differences in threshold cycle number are used to quantify the relative amount of PCR target contained within each tube. Assuming that each reaction functions at 100% PCR efficiency, a difference of one Ct represents a two-fold difference in the amount of starting template. The fluorescence value can be used in conjunction with a standard curve to determine the amount of amplification product present.

### Non-Probe-Based Detection Methods

A variety of options are available for measuring the amplification products as they are formed. One method utilizes labels, such as dyes, which only bind to double stranded DNA. In this type of approach, amplification product (which is double stranded) binds dye molecules in solution to form a complex. With the appropriate dyes, it is possible to distinguish between dye molecules free in solution and dye molecules bound to amplification product. For example, certain dyes fluoresce only when bound to amplification product. Examples of dyes which can be used in methods of this general type include, but are not limited to, Syber Green.TM. and Pico Green from Molecular Probes, Inc. of Eugene, Oreg., ethidium bromide, propidium iodide, chromomycin, acridine orange, Hoechst 33258, Toto-1, Yoyo-1, DAPI (4',6-diamidino-2-phenylindole hydrochloride).

Another real time detection technique measures alteration in energy fluorescence energy transfer between fluorophors conjugated with PCR primers [Livak, (1995)].

### Probe-Based Detection Methods

These detection methods involve some alteration to the structure or conformation of a probe hybridized to the locus between the amplification primer pair. In some instances, the alteration is caused by the template-dependent extension catalyzed by a nucleic acid polymerase during the amplification process. The alteration generates a detectable signal which is an indirect measure of the amount of amplification product formed.

For example, some methods involve the degradation or digestion of the probe during the extension reaction. These methods are a consequence of the 5'-3' nuclease activity associated with some nucleic acid polymerases. Polymerases having this activity cleave mononucleotides or small oligonucleotides from an oligonucleotide probe annealed to its complementary sequence located within the locus.

The 3' end of the upstream primer provides the initial binding site for the nucleic acid polymerase. As the polymerase catalyzes extension of the upstream primer and encounters the bound probe, the nucleic acid polymerase displaces a portion of the 5' end of the probe and through its nuclease activity cleaves mononucleotides or oligonucleotides from the probe.

The upstream primer and the probe can be designed such that they anneal to the complementary strand in close proximity to one another. In fact, the 3' end of the upstream primer and the 5' end of the probe may abut one another. In this situation, extension of the upstream primer is not necessary in order for the nucleic acid polymerase to begin cleaving the probe. In the case in which intervening nucleotides separate the upstream primer and the probe, extension of the primer is necessary before the nucleic acid polymerase encounters the 5' end of the probe. Once contact occurs and polymerization continues, the 5'-3' exonuclease activity of the nucleic acid polymerase begins cleaving mononucleotides or oligonucleotides from the 5' end of the probe. Digestion of the probe continues until the remaining portion of the probe dissociates from the complementary strand.

In solution, the two end sections can hybridize with each other to form a hairpin loop. In this conformation, the reporter and quencher dye are in sufficiently close proximity that fluorescence from the reporter dye is effectively quenched by the quencher dye. Hybridized probe, in contrast, results in a linearized conformation in which the extent of quenching is decreased. Thus, by monitoring emission changes for the two dyes, it is possible to indirectly monitor the formation of amplification product.

### Probes

The labeled probe is selected so that its sequence is substantially complementary to a segment of the test locus or a reference locus. As indicated above, the nucleic acid site to which the probe binds should be located between the primer binding sites for the upstream and downstream amplification primers.

### Primers

The primers used in the amplification are selected so as to be capable of hybridizing to sequences at flanking regions of the locus being amplified. The primers are chosen to have at least substantial complementarity with the different strands of the nucleic acid being amplified. When a probe is utilized to detect the formation of amplification products, the primers are selected in such that they flank the probe, i.e. are located upstream and downstream of the probe.

The primer must have sufficient length so that it is capable of priming the synthesis of extension products in the presence of an agent for polymerization. The length and composition of the primer depends on many parameters, including, for example, the temperature at which the annealing reaction is conducted, proximity of the probe binding site to that of the primer, relative concentrations of the primer and probe and the particular nucleic acid composition of the probe. Typically the primer includes 15-30 nucleotides. However, the length of the primer may be more or less depending on the complexity of the primer binding site and the factors listed above.

### Labels for Probes and Primers

The labels used for labeling the probes or primers of the current invention and which can provide the signal corresponding to the quantity of amplification product can take a variety of forms. As indicated above with regard to the 5' fluorogenic nuclease method, a fluorescent signal is one signal which can be measured. However, measurements may also be made, for example, by monitoring radioactivity, colorimetry, absorption, magnetic parameters, or enzymatic activity. Thus, labels which can be employed include, but are not limited to, fluorophors, chromophores, radioactive isotopes, electron dense reagents, enzymes, and ligands having specific binding partners (e.g., biotin-avidin).

Monitoring changes in fluorescence is a particularly useful way to monitor the accumulation of amplification products. A number of labels useful for attachment to probes or primers are commercially available including fluorescein and various fluorescein derivatives such as FAM, HEX, TET and JOE (all which are available from Applied Biosystems, Foster City, Calif.); lucifer yellow, and coumarin derivatives.

Labels may be attached to the probe or primer using a variety of techniques and can be attached at the 5' end, and/or the 3' end and/or at an internal nucleotide. The label can also be attached to spacer arms of various sizes which are attached to the probe or primer. These spacer arms are useful for obtaining a desired distance between multiple labels attached to the probe or primer.

In some instances, a single label may be utilized; whereas, in other instances, such as with the 5' fluorogenic nuclease assays for example, two or more labels are attached to the probe. In cases wherein the probe includes multiple labels, it is generally advisable to maintain spacing between the labels which is sufficient to permit separation of the labels during digestion of the probe through the 5'-3' nuclease activity of the nucleic acid polymerase.

### Microarray

Nucleic acid arrays that have been used in the present invention are those that are commercially available from Affymetrix (Santa Clara, Calif.) under the brand name GeneChip Human Genome U133 Plus 2.0 Array.® or Rat Genome U230 plus 2.0 Array respectively which represents the complete coverage of the Human Genome U133 Set plus 9921 probe sets representing approximately 6,500 new genes (with a total of approximately 56 000 transcripts) or the Rat Genome respectively. Affymetrix (Santa Clara, Calif.) GeneChip technology platform which consists of high-density microarrays and tools to help process and analyze those arrays, including standardized assays and reagents, instrumentation, and data management and analysis tools.

GeneChip microarrays consist of small DNA fragments (referred to as probes), chemically synthesized at specific locations on a coated quartz surface. By extracting and labeling nucleic acids from experimental samples, and then hybridizing those prepared samples to the array, the amount of label can be monitored enabling a measurement of gene regulation

The GeneChip human genome arrays include a set of human maintenance genes to facilitate the normalization and scaling of array experiments and to perform data comparison. This set of normalization genes shows consistent levels of expression over a diverse set of tissues.

### Patients Exhibiting Symptoms of Disease

A number of diseases are associated with changes in the copy number of a certain gene. For patients having symptoms of a disease, the real-time PCR method can be used to determine if the patient has copy number alterations which are known to be linked with diseases that are associated with the symptoms the patient has.

### CTGF expression

### CTGF fusion proteins

Fusion proteins are useful for generating antibodies against CTGF polypeptides and for use in various assay systems. For example, fusion proteins can be used to identify proteins which interact with portions of CTGF polypeptides. Protein affinity chromatography or library-based assays for protein-protein interactions, such as the yeast two-hybrid or phage display systems, can be used for this purpose. Such methods are well known in the art and also can be used as drug screens.

A CTGF fusion protein comprises two polypeptide segments fused together by means of a peptide bond. The first polypeptide segment can comprise at least 54, 75, 100, 125, 139, 150, 175, 200, 225, 250, 275, 300, 325 or 350 contiguous amino acids of SEQ ID NO: 3 or 4 or of a variant, such as those described above. The first polypeptide segment also can comprise full-length CTGF.

The second polypeptide segment can be a full-length protein or a protein fragment. Proteins commonly used in fusion protein construction include, but are not limited to β galactosidase, β-glucuronidase, green fluorescent protein (GFP), autofluorescent proteins, including blue fluorescent protein (BFP), glutathione-S-transferase (GST), luciferase, horseradish peroxidase (HRP), and chloramphenicol acetyltransferase (CAT). Additionally, epitope tags are used in fusion protein constructions, including histidine (His) tags, FLAG tags, influenza hemagglutinin (HA) tags, Myc tags, VSV-G tags, and thioredoxin (Trx) tags. Other fusion constructions can include maltose binding protein (MBP), S-tag, Lex a DNA binding domain (DBD) fusions, GAL4 DNA binding domain fusions, and herpes simplex virus (HSV) BP16 protein fusions. A fusion protein also can be engineered to contain a cleavage site located adjacent to the CTGF.

### Preparation of Polynucleotides

A naturally occurring CTGF polynucleotide can be isolated free of other cellular components such as membrane components, proteins, and lipids. Polynucleotides can be made by a cell and isolated using standard nucleic acid purification techniques, or synthesized using an amplification technique, such as the polymerase chain reaction (PCR), or by using an automatic synthesizer. Methods for isolating polynucleotides are routine and are known in the art. Any such technique for obtaining a polynucleotide can be used to obtain isolated CTGF polynucleotides. For example, restriction enzymes and probes can be used to isolate polynucleotide fragments which comprise CTGF nucleotide sequences. Isolated polynucleotides are in preparations which are free or at least 70, 80, or 90% free of other molecules.

CTGF cDNA molecules can be made with standard molecular biology techniques, using CTGF mRNA as a template. CTGF cDNA molecules can thereafter be replicated using molecular biology techniques known in the art. An amplification technique, such as PCR, can be used to obtain additional copies of polynucleotides of the invention, using either human genomic DNA or cDNA as a template.

Alternatively, synthetic chemistry techniques can be used to synthesizes CTGF polynucleotides. The degeneracy of the genetic code allows alternate nucleotide sequences to be synthesized which will encode CTGF having, for example, an amino acid sequence shown in SEQ ID NO: 2 or a biologically active variant thereof.

### Extending Polynucleotides

Various PCR-based methods can be used to extend nucleic acid sequences encoding human CTGF, for example to detect upstream sequences of CTGF gene such as promoters and regulatory elements. For example, restriction-site PCR uses universal primers to retrieve unknown sequence adjacent to a known locus. Genomic DNA is first amplified in the presence of a primer to a linker sequence and a primer specific to the known region. The amplified sequences are then subjected to a second round of PCR with the same linker primer and another specific primer internal to the first one. Products of each round of PCR are transcribed with an appropriate RNA polymerase and sequenced using reverse transcriptase.

Inverse PCR also can be used to amplify or extend sequences using divergent primers based on a known region. Primers can be designed using commercially available software, such as OLIGO 4.06 Primer Analysis software (National Biosciences Inc., Plymouth, Minn.), to be 22-30 nucleotides in length, to have a GC content of 50% or more, and to anneal to the target sequence at temperatures about 68-72°C. The method uses several restriction enzymes to generate a suitable fragment in the known region of a gene. The fragment is then circularized by intramolecular ligation and used as a PCR template.

Another method which can be used is capture PCR, which involves PCR amplification of DNA fragments adjacent to a known sequence in human and yeast artificial chromosome DNA. In this method, multiple restriction enzyme digestions and ligations also can be used to place an engineered double-stranded sequence into an unknown fragment of the DNA molecule before performing PCR.

When screening for full-length cDNAs, it is preferable to use libraries that have been size-selected to include larger cDNAs. Randomly-primed libraries are preferable, in that they will contain more sequences which contain the 5' regions of genes. Use of a randomly primed library may be especially preferable for situations in which an oligo d(T) library does not yield a full-length cDNA. Genomic libraries can be useful for extension of sequence into 5' non-transcribed regulatory regions.

Commercially available capillary electrophoresis systems can be used to analyze the size or confirm the nucleotide sequence of PCR or sequencing products. For example, capillary sequencing can employ flowable polymers for electrophoretic separation, four different fluorescent dyes (one for each nucleotide) which are laser activated, and detection of the emitted wavelengths by a charge coupled device camera. Output/light intensity can be converted to electrical signal using appropriate equipment and software *(e.g.,* GENOTYPER and Sequence NAVIGATOR, Perkin Elmer), and the entire process from loading of samples to computer analysis and electronic data display can be computer controlled. Capillary electrophoresis is especially preferable for the sequencing of small pieces of DNA which might be present in limited amounts in a particular sample.

### Obtaining Polypeptides

CTGF can be obtained, for example, by purification from human cells, by expression of CTGF polynucleotides, or by direct chemical synthesis.

### Protein Purification

CTGF can be purified from any human cell which expresses the enzyme, including those which have been transfected with expression constructs which express CTGF. A purified CTGF is separated from other compounds which normally associate with CTGF in the cell, such as certain proteins, carbohydrates, or lipids, using methods well-known in the art. Such methods include, but are not limited to, size exclusion chromatography, ammonium sulfate fractionation, ion exchange chromatography, affinity chromatography, and preparative gel electrophoresis.

### Expression of CTGF Polynucleotides

To express CTGF, CTGF polynucleotides can be inserted into an expression vector which contains the necessary elements for the transcription and translation of the inserted coding sequence. Methods which are well known to those skilled in the art can be used to construct expression vectors containing sequences encoding CTGF and appropriate transcriptional and translational control elements. These methods include *in vitro* recombinant DNA techniques, synthetic techniques, and *in vivo* genetic recombination.

A variety of expression vector/host systems can be utilized to contain and express sequences encoding CTGF. These include, but are not limited to, microorganisms, such as bacteria transformed with recombinant bacteriophage, plasmid, or cosmid DNA expression vectors; yeast transformed with yeast expression vectors, insect cell systems infected with virus expression vectors (*e.g.,* baculovirus), plant cell systems transformed with virus expression vectors (*e.g.,* cauliflower mosaic virus, CaMV; tobacco mosaic virus, TMV) or with bacterial expression vectors (*e.g*., Ti or pBR322 plasmids), or animal cell systems.

The control elements or regulatory sequences are those non-translated regions of the vector - enhancers, promoters, 5' and 3' untranslated regions -- which interact with host cellular proteins to carry out transcription and translation. Such elements can vary in their strength and specificity. Depending on the vector system and host utilized, any number of suitable transcription and translation elements, including constitutive and inducible promoters, can be used. For example, when cloning in bacterial systems, inducible promoters such as the hybrid lacZ promoter of the BLUESCRIPT phagemid (Stratagene, LaJolla, Calif.) or pSPORT1 plasmid (Life Technologies) and the like can be used. The baculovirus polyhedrin promoter can be used in insect cells. Promoters or enhancers derived from the genomes of plant cells *(e.g.,* heat shock, RUBISCO, and storage protein genes) or from plant viruses (*e.g*., viral promoters or leader sequences) can be cloned into the vector. In mammalian cell systems, promoters from mammalian genes or from mammalian viruses are preferable. If it is necessary to generate a cell line that contains multiple copies of a nucleotide sequence encoding CTGF, vectors based on SV40 or EBV can be used with an appropriate selectable marker.

### Bacterial and Yeast Expression Systems

In bacterial systems, a number of expression vectors can be selected. For example, when a large quantity of CTGF is needed for the induction of antibodies, vectors which direct high level expression of fusion proteins that are readily purified can be used. Such vectors include, but are not limited to, multifunctional *E. coli* cloning and expression vectors such as BLUESCRIPT (Stratagene). In a BLUESCRIPT vector, a sequence encoding CTGF can be ligated into the vector in frame with sequences for the amino-terminal Met and the subsequent 7 residues of β-galactosidase so that a hybrid protein is produced. pIN vectors or pGEX vectors (Promega, Madison, Wis.) also can be used to express foreign polypeptides as fusion proteins with glutathione S-transferase (GST). In general, such fusion proteins are soluble and can easily be purified from lysed cells by adsorption to glutathione-agarose beads followed by elution in the presence of free glutathione. Proteins made in such systems can be designed to include heparin, thrombin, or factor Xa protease cleavage sites so that the cloned polypeptide of interest can be released from the GST moiety at will.

### Plant and Insect Expression Systems

If plant expression vectors are used, the expression of sequences encoding CTGF can be driven by any of a number of promoters. For example, viral promoters such as the 35S and 19S promoters of CaMV can be used alone or in combination with the omega leader sequence from TMV. Alternatively, plant promoters such as the small subunit of RUBISCO or heat shock promoters can be used. These constructs can be introduced into plant cells by direct DNA transformation or by pathogen-mediated transfection.

An insect system also can be used to express CTGF. For example, in one such system *Autographa californica* nuclear polyhedrosis virus (AcNPV) is used as a vector to express foreign genes in *Spodoptera frugiperda* cells or in *Trichoplusia* larvae. Sequences encoding CTGF can be cloned into a non-essential region of the virus, such as the polyhedrin gene, and placed under control of the polyhedrin promoter. Successful insertion of CTGF will render the polyhedrin gene inactive and produce recombinant virus lacking coat protein. The recombinant viruses can then be used to infect *S*. *frugiperda cells* or *Trichoplusia* larvae in which CTGF can be expressed.

### Mammalian Expression Systems

A number of viral-based expression systems can be used to express CTGF in mammalian host cells. For example, if an adenovirus is used as an expression vector, sequences encoding CTGF can be ligated into an adenovirus transcription/translation complex comprising the late promoter and tripartite leader sequence. Insertion in a non-essential E1 or E3 region of the viral genome can be used to obtain a viable virus which is capable of expressing CTGF in infected host cells [Engelhard, (1994)]. If desired, transcription enhancers, such as the Rous sarcoma virus (RSV) enhancer, can be used to increase expression in mammalian host cells.

Human artificial chromosomes (HACs) also can be used to deliver larger fragments of DNA than can be contained and expressed in a plasmid. HACs of 6M to 10M are constructed and delivered to cells via conventional delivery methods (*e.g*., liposomes, polycationic amino polymers, or vesicles). Specific initiation signals also can be used to achieve more efficient translation of sequences encoding CTGF. Such signals include the ATG initiation codon and adjacent sequences. In cases where sequences encoding CTGF, its initiation codon, and upstream sequences are inserted into the appropriate expression vector, no additional transcriptional or translational control signals may be needed. However, in cases where only coding sequence, or a fragment thereof, is inserted, exogenous translational control signals (including the ATG initiation codon) should be provided. The initiation codon should be in the correct reading frame to ensure translation of the entire insert. Exogenous translational elements and initiation codons can be of various origins, both natural and synthetic.

### Host Cells

A host cell strain can be chosen for its ability to modulate the expression of the inserted sequences or to process the expressed CTGF in the desired fashion. Such modifications of the polypeptide include, but are not limited to, acetylation, carboxylation, glycosylation, phosphorylation, lipidation, and acylation. Post-translational processing which cleaves a "prepro" form of the polypeptide also can be used to facilitate correct insertion, folding and/or function. Different host cells which have specific cellular machinery and characteristic mechanisms for post-translational activities (*e.g*., CHO, HeLa, MDCK, HEK293, and WI38), are available from the American Type Culture Collection (ATCC; 10801 University Boulevard, Manassas, VA 20110-2209) and can be chosen to ensure the correct modification and processing of the foreign protein.

Stable expression is preferred for long-term, high-yield production of recombinant proteins. For example, cell lines which stably express CTGF can be transformed using expression vectors which can contain viral origins of replication and/or endogenous expression elements and a selectable marker gene on the same or on a separate vector. Following the introduction of the vector, cells can be allowed to grow for 1-2 days in an enriched medium before they are switched to a selective medium. The purpose of the selectable marker is to confer resistance to selection, and its presence allows growth and recovery of cells which successfully express the introduced CTGF sequences. Resistant clones of stably transformed cells can be proliferated using tissue culture techniques appropriate to the cell type. Any number of selection systems can be used to recover transformed cell lines. These include, but are not limited to, the herpes simplex virus thymidine kinase [Logan, (1984)] and adenine phosphoribosyltransferase [Wigler, (1977)] genes which can be employed in *tk⁻* or *aprt⁻* cells, respectively. Also, antimetabolite, antibiotic, or herbicide resistance can be used as the basis for selection. For example, *dhfr* confers resistance to methotrexate [Lowy, (1980)], *npt* confers resistance to the aminoglycosides, neomycin and G-418 [Wigler, (1980)], and *als* and *pat* confer resistance to chlorsulfuron and phosphinotricin acetyltransferase, respectively [Colbere-Garapin, 1981]. Additional selectable genes have been described. For example, *trpB* allows cells to utilize indole in place of tryptophan, or *hisD,* which allows cells to utilize histinol in place of histidine. Visible markers such as anthocyanins, β-glucuronidase and its substrate GUS, and luciferase and its substrate luciferin, can be used to identify transformants and to quantify the amount of transient or stable protein expression attributable to a specific vector system

### Detecting Polypeptide Expression

Although the presence of marker gene expression suggests that a CTGF polynucleotide is also present, its presence and expression may need to be confirmed. For example, if a sequence encoding CTGF is inserted within a marker gene sequence, transformed cells containing sequences which encode CTGF can be identified by the absence of marker gene function. Alternatively, a marker gene can be placed in tandem with a sequence encoding CTGF under the control of a single promoter. Expression of the marker gene in response to induction or selection usually indicates expression of CTGF polynucleotide.

Alternatively, host cells which contain a CTGF polynucleotide and which express CTGF can be identified by a variety of procedures known to those of skill in the art. These procedures include, but are not limited to, DNA-DNA or DNA-RNA hybridizations and protein bioassay or immunoassay techniques which include membrane, solution, or chip-based technologies for the detection and/or quantification of nucleic acid or protein. For example, the presence of a polynucleotide sequence encoding CTGF can be detected by DNA-DNA or DNA-RNA hybridization or amplification using probes or fragments or fragments of polynucleotides encoding CTGF. Nucleic acid amplification-based assays involve the use of oligonucleotides selected from sequences encoding CTGF to detect transformants which contain a CTGF polynucleotide.

A variety of protocols for detecting and measuring the expression of CTGF, using either polyclonal or monoclonal antibodies specific for the polypeptide, are known in the art. Examples include enzyme-linked immunosorbent assay (ELISA), radioimmunoassay (RIA), and fluorescence activated cell sorting (FACS). A two-site, monoclonal-based immunoassay using Monoclonal antibodies reactive to two non-interfering epitopes on CTGF can be used, or a competitive binding assay can be employed.

A wide variety of labels and conjugation techniques are known by those skilled in the art and can be used in various nucleic acid and amino acid assays. Means for producing labeled hybridization or PCR probes for detecting sequences related to polynucleotides encoding CTGF include oligolabeling, nick translation, end-labeling, or PCR amplification using a labeled nucleotide. Alternatively, sequences encoding CTGF can be cloned into a vector for the production of an mRNA probe. Such vectors are known in the art, are commercially available, and can be used to synthesize RNA probes *in vitro* by addition of labeled nucleotides and an appropriate RNA polymerase such as T7, T3, or SP6. These procedures can be conducted using a variety of commercially available kits (Amersham Pharmacia Biotech, Promega, and US Biochemical). Suitable reporter molecules or labels which can be used for ease of detection include radionuclides, enzymes, and fluorescent, chemiluminescent, or chromogenic agents, as well as substrates, cofactors, inhibitors, magnetic particles, and the like.

### Expression and Purification of Polypeptides

Host cells transformed with CTGF polynucleotides can be cultured under conditions suitable for the expression and recovery of the protein from cell culture. The polypeptide produced by a transformed cell can be secreted or contained intracellularly depending on the sequence and/or the vector used. As will be understood by those of skill in the art, expression vectors containing CTGF polynucleotides can be designed to contain signal sequences which direct secretion of soluble CTGF through a prokaryotic or eukaryotic cell membrane or which direct the membrane insertion of membrane-bound CTGF.

As discussed above, other constructions can be used to join a sequence encoding CTGF to a nucleotide sequence encoding a polypeptide domain which will facilitate purification of soluble proteins. Such purification facilitating domains include, but are not limited to, metal chelating peptides such as histidine-tryptophan modules that allow purification on immobilized metals, protein A domains that allow purification on immobilized immunoglobulin, and the domain utilized in the FLAGS extension/affinity purification system (Immunex Corp., Seattle, Wash.). Inclusion of cleavable linker sequences such as those specific for Factor XA or enterokinase (Invitrogen, San Diego, CA) between the purification domain and CTGF also can be used to facilitate purification. One such expression vector provides for expression of a fusion protein containing CTGF and 6 histidine residues preceding a thioredoxin or an enterokinase cleavage site. The histidine residues facilitate purification by IMAC (immobilized metal ion affinity chromatography) Maddox, (1983)], while the enterokinase cleavage site provides a means for purifying CTGF from the fusion protein [Porath, (1992)].

### Chemical Synthesis

Sequences encoding CTGF can be synthesized, in whole or in part, using chemical methods well known in the art. Alternatively, CTGF itself can be produced using chemical methods to synthesize its amino acid sequence, such as by direct peptide synthesis using solid-phase techniques. Protein synthesis can either be performed using manual techniques or by automation. Automated synthesis can be achieved, for example, using Applied Biosystems 431A Peptide Synthesizer (Perkin Elmer). Optionally, fragments of CTGF can be separately synthesized and combined using chemical methods to produce a full-length molecule.

The newly synthesized peptide can be substantially purified by preparative high performance liquid chromatography. The composition of a synthetic CTGF can be confirmed by amino acid analysis or sequencing. Additionally, any portion of the amino acid sequence of CTGF can be altered during direct synthesis and/or combined using chemical methods with sequences from other proteins to produce a variant polypeptide or a fusion protein.

### Production of Altered Polypeptides

As will be understood by those of skill in the art, it may be advantageous to produce CTGF polynucleotides possessing non-naturally occurring codons. For example, codons preferred by a particular prokaryotic or eukaryotic host can be selected to increase the rate of protein expression or to produce an RNA transcript having desirable properties, such as a half-life which is longer than that of a transcript generated from the naturally occurring sequence.

The nucleotide sequences referred to herein can be engineered using methods generally known in the art to alter CTGF polynucleotides for a variety of reasons, including but not limited to, alterations which modify the cloning, processing, and/or expression of the polypeptide or mRNA product. DNA shuffling by random fragmentation and PCR reassembly of gene fragments and synthetic oligonucleotides can be used to engineer the nucleotide sequences. For example, site-directed mutagenesis can be used to insert new restriction sites, alter glycosylation patterns, change codon preference, produce splice variants, introduce mutations, and so forth.

### CTGF Analogs

One general class of CTGF analogs are variants having an amino acid sequence that is a mutation of the amino acid sequence disclosed herein. Another general class of CTGF analogs is provided by anti-idiotype antibodies, and fragments thereof, as described below. Moreover, recombinant antibodies comprising anti-idiotype variable domains can be used as analogs (see, for example, [Monfardini et al., (1996)]). Since the variable domains of anti-idiotype CTGF antibodies mimic CTGF, these domains can provide CTGF enzymatic activity. Methods of producing anti-idiotypic catalytic antibodies are known to those of skill in the art [Joron et al., (1992), Friboulet et al. (1994), Avalle et al., (1998)].

Another approach to identifying CTGF analogs is provided by the use of combinatorial libraries. Methods for constructing and screening phage display and other combinatorial libraries are provided, for example, by [Kay et al., Phage Display of Peptides and Proteins (Academic Press 1996), U.S. 5,783,384, U.S. 5,747,334, and U.S. 5,723,323.

### Antibodies

Any type of antibody known in the art can be generated to bind specifically to an epitope of CTGF.

"Antibody" as used herein includes intact immunoglobulin molecules, as well as fragments thereof, such as Fab, F(ab')₂, and Fv, which are capable of binding an epitope of CTGF. Typically, at least 6, 8, 10, or 12 contiguous amino acids are required to form an epitope. However, epitopes which involve non-contiguous amino acids may require more, e.g., at least 15, 25, or 50 amino acid. An antibody which specifically binds to an epitope of CTGF can be used therapeutically, as well as in immunochemical assays, such as Western blots, ELISAs, radioimmunoassays, immunohistochemical assays, immunoprecipitations, or other immunochemical assays known in the art. Various immunoassays can be used to identify antibodies having the desired specificity. Numerous protocols for competitive binding or immunoradiometric assays are well known in the art. Such immunoassays typically involve the measurement of complex formation between an immunogen and an antibody which specifically binds to the CTGF immunogen.

Typically, an antibody which specifically binds to CTGF provides a detection signal at least 5-, 10-, or 20-fold higher than a detection signal provided with other proteins when used in an immunochemical assay. Preferably, antibodies which specifically bind to CTGF do not detect other proteins in immunochemical assays and can immunoprecipitate CTGF from solution.

CTGF can be used to immunize a mammal, such as a mouse, rat, rabbit, guinea pig, monkey, or human, to produce polyclonal antibodies. If desired, CTGF can be conjugated to a carrier protein, such as bovine serum albumin, thyroglobulin, and keyhole limpet hemocyanin. Depending on the host species, various adjuvants can be used to increase the immunological response. Such adjuvants include, but are not limited to, Freund's adjuvant, mineral gels *(e.g.,* aluminum hydroxide), and surface active substances (e.g., lysolecithin, pluronic polyols, polyanions, peptides, oil emulsions, keyhole limpet hemocyanin, and dinitrophenol). Among adjuvants used in humans, BCG (*bacilli Calmette-Guerin*) and *Corynebacterium parvum* are especially useful.

Monoclonal antibodies which specifically bind to CTGF can be prepared using any technique which provides for the production of antibody molecules by continuous cell lines in culture. These techniques include, but are not limited to, the hybridoma technique, the human B-cell hybridoma technique, and the EBV-hybridoma technique [Roberge, (1995)].

In addition, techniques developed for the production of "chimeric antibodies", the splicing of mouse antibody genes to human antibody genes to obtain a molecule with appropriate antigen specificity and biological activity, can be used. Monoclonal and other antibodies also can be "humanized" to prevent a patient from mounting an immune response against the antibody when it is used therapeutically. Such antibodies may be sufficiently similar in sequence to human antibodies to be used directly in therapy or may require alteration of a few key residues. Sequence differences between rodent antibodies and human sequences can be minimized by replacing residues which differ from those in the human sequences by site directed mutagenesis of individual residues or by grating of entire complementarity determining regions. Antibodies which specifically bind to CTGF can contain antigen binding sites which are either partially or fully humanized, as disclosed in U.S. 5,565,332.

Alternatively, techniques described for the production of single chain antibodies can be adapted using methods known in the art to produce single chain antibodies which specifically bind to CTGF. Antibodies with related specificity, but of distinct idiotypic composition, can be generated by chain shuffling from random combinatorial immunoglobin libraries. Single-chain antibodies also can be constructed using a DNA amplification method, such as PCR, using hybridoma cDNA as a template. Single-chain antibodies can be mono- or bispecific, and can be bivalent or tetravalent. Construction of tetravalent, bispecific single-chain antibodies is taught. A nucleotide sequence encoding a single-chain antibody can be constructed using manual or automated nucleotide synthesis, cloned into an expression construct using standard recombinant DNA methods, and introduced into a cell to express the coding sequence, as described below. Alternatively, single-chain antibodies can be produced directly using, for example, filamentous phage technology.

Antibodies which specifically bind to CTGF also can be produced by inducing *in vivo* production in the lymphocyte population or by screening immunoglobulin libraries or panels of highly specific binding reagents. Other types of antibodies can be constructed and used therapeutically in methods of the invention. For example, chimeric antibodies can be constructed as disclosed in WO 93/03151. Binding proteins which are derived from immunoglobulins and which are multivalent and multispecific, such as the "diabodies" described in WO 94/13804, also can be prepared.

Antibodies according to the invention can be purified by methods well known in the art. For example, antibodies can be affinity purified by passage over a column to which CTGF is bound. The bound antibodies can then be eluted from the column using a buffer with a high salt concentration.

### Antisense Oligonucleotides

Antisense oligonucleotides are nucleotide sequences which are complementary to a specific DNA or RNA sequence. Once introduced into a cell, the complementary nucleotides combine with natural sequences produced by the cell to form complexes and block either transcription or translation. Preferably, an antisense oligonucleotide is at least 11 nucleotides in length, but can be at least 12, 15, 20, 25, 30, 35, 40, 45, or 50 or more nucleotides long. Longer sequences also can be used. Antisense oligonucleotide molecules can be provided in a DNA construct and introduced into a cell as described above to decrease the level of CTGF gene products in the cell.

Antisense oligonucleotides can be deoxyribonucleotides, ribonucleotides, or a combination of both. Oligonucleotides can be synthesized manually or by an automated synthesizer, by covalently linking the 5' end of one nucleotide with the 3' end of another nucleotide with non-phosphodiester internucleotide linkages such alkylphosphonates, phosphorothioates, phosphorodithioates, alkylphosphonothioates, alkylphosphonates, phosphoramidates, phosphate esters, carbamates, acetamidate, carboxymethyl esters, carbonates, and phosphate triesters.

Modifications of CTGF gene expression can be obtained by designing antisense oligonucleotides which will form duplexes to the control, 5', or regulatory regions of the CTGF gene. Oligonucleotides derived from the transcription initiation site, *e.g.,* between positions -10 and +10 from the start site, are preferred. Similarly, inhibition can be achieved using "triple helix" base-pairing methodology. Triple helix pairing is useful because it causes inhibition of the ability of the double helix to open sufficiently for the binding of polymerases, transcription factors, or chaperons. Therapeutic advances using triplex DNA have been described in the literature [Nicholls, (1993)]. An antisense oligonucleotide also can be designed to block translation of mRNA by preventing the transcript from binding to ribosomes.

Precise complementarity is not required for successful complex formation between an antisense oligonucleotide and the complementary sequence of a CTGF polynucleotide. Antisense oligonucleotides which comprise, for example, 2, 3, 4, or 5 or more stretches of contiguous nucleotides which are precisely complementary to a CTGF polynucleotide, each separated by a stretch of contiguous nucleotides which are not complementary to adjacent CTGF nucleotides, can provide sufficient targeting specificity for CTGF mRNA. Preferably, each stretch of complementary contiguous nucleotides is at least 4, 5, 6, 7, or 8 or more nucleotides in length. Non-complementary intervening sequences are preferably 1, 2, 3, or 4 nucleotides in length. One skilled in the art can easily use the calculated melting point of an antisense-sense pair to determine the degree of mismatching which will be tolerated between a particular antisense oligonucleotide and a particular CTGF polynucleotide sequence. Antisense oligonucleotides can be modified without affecting their ability to hybridize to a CTGF polynucleotide. These modifications can be internal or at one or both ends of the antisense molecule. For example, internucleoside phosphate linkages can be modified by adding cholesteryl or diamine moieties with varying numbers of carbon residues between the amino groups and terminal ribose. Modified bases and/or sugars, such as arabinose instead of ribose, or a 3', 5'-substituted oligonucleotide in which the 3' hydroxyl group or the 5' phosphate group are substituted, also can be employed in a modified antisense oligonucleotide. These modified oligonucleotides can be prepared by methods well known in the art.

### Ribozymes

Ribozymes are RNA molecules with catalytic activity [Uhlmann, (1987)]. Ribozymes can be used to inhibit gene function by cleaving an RNA sequence, as is known in the art. The mechanism of ribozyme action involves sequence-specific hybridization of the ribozyme molecule to complementary target RNA, followed by endonucleolytic cleavage. Examples include engineered hammerhead motif ribozyme molecules that can specifically and efficiently catalyze endonucleolytic cleavage of specific nucleotide sequences. The coding sequence of a CTGF polynucleotide can be used to generate ribozymes which will specifically bind to mRNA transcribed from a CTGF polynucleotide. Methods of designing and constructing ribozymes which can cleave other RNA molecules in trans in a highly sequence specific manner have been developed and described in the art. For example, the cleavage activity of ribozymes can be targeted to specific RNAs by engineering a discrete "hybridization" region into the ribozyme. The hybridization region contains a sequence complementary to the target RNA and thus specifically hybridizes with the target RNA.

Specific ribozyme cleavage sites within a CTGF RNA target can be identified by scanning the target molecule for ribozyme cleavage sites which include the following sequences: GUA, GUU, and GUC. Once identified, short RNA sequences of between 15 and 20 ribonucleotides corresponding to the region of the target RNA containing the cleavage site can be evaluated for secondary structural features which may render the target inoperable. Suitability of candidate CTGF RNA targets also can be evaluated by testing accessibility to hybridization with complementary oligonucleotides using ribonuclease protection assays. The nucleotide sequences shown in SEQ ID NO: 1 and its complement provide sources of suitable hybridization region sequences. Longer complementary sequences can be used to increase the affinity of the hybridization sequence for the target. The hybridizing and cleavage regions of the ribozyme can be integrally related such that upon hybridizing to the target RNA through the complementary regions, the catalytic region of the ribozyme can cleave the target.

Ribozymes can be introduced into cells as part of a DNA construct. Mechanical methods, such as microinjection, liposome-mediated transfection, electroporation, or calcium phosphate precipitation, can be used to introduce a ribozyme-containing DNA construct into cells in which it is desired to decrease CTGF expression. Alternatively, if it is desired that the cells stably retain the DNA construct, the construct can be supplied on a plasmid and maintained as a separate element or integrated into the genome of the cells, as is known in the art. A ribozyme-encoding DNA construct can include transcriptional regulatory elements, such as a promoter element, an enhancer or UAS element, and a transcriptional terminator signal, for controlling transcription of ribozymes in the cells (U.S. 5,641,673). Ribozymes also can be engineered to provide an additional level of regulation, so that destruction of mRNA occurs only when both a ribozyme and a target gene are induced in the cells.

### Assays for CTGF and CTGF bioactivity

The presence of CTGF protein/DNA/RNA in tissue and body liquids is determined by standard immune assay technologies like ELISA, RIA etc or quantitative Real Time PCR (qRT PCR).

CTGF ELISA. : CTGF levels in culture supernatants, blood plasma, and urine are measured using a sandwich ELISA that detects whole CTGF and the NH2-terminal fragment of CTGF that persists in body fluids and cell culture supernatants after proteolytic cleavage of the hinge domain.

CTGF Real Time PCR: For confirmation of CTGF mRNA expression qRT-PCR is used. cDNA obtained by reverse transcription of DNase-treated total RNA from each sample using random hexamer priming in 50 micro liter reactions according to the manufacturer's recommendations (TaqMan reverse transcription reagent kit; Applied Biosystems, Foster City, CA). qRT-PCR is proceeded using the Applied Biosystems Prism 7900HT sequence detection system. Expression values are normalized to human glyceraldehyde-3-phosphate dehydrogenase. qRT-PCR primers are designed using Primer Express version 2.0.0 (Applied Biosystems) and tested to confirm appropriate product size and optimal concentrations.

CTGF binding to alpha v beta 3: Platelets are diluted in binding buffer (consisting of 0.05 M HEPES, pH 7.4, and 5 mM MnCl2) at a concentration of 1.5 × 10⁶ human platelets or 5 × 10⁶ rat platelets/100 µl. Platelets (100 µl) are incubated with increasing concentrations of 125I labeled CTGF in a total volume of 250 µl of binding buffer. After 90-min incubation at room temperature, the samples are filtered on 34 glass fiber paper (Schleicher & Schuell, Keene, NH), then washed three times with 3 ml of 0.05 M Tris-HCl, pH 7.4, and 0.154 M NaCl on a 30-well Brandel cell harvester (Gaithersburg, MD). The filters are presoaked for 1 h in washing buffer containing 5% dry skim milk (Carnation, Nestlé, Don Mills, Ontario, Canada) to reduce nonspecific adsorption. Radioactivity is counted in a gamma counter with an efficiency of 80%. Competition curves are analyzed by the Hill equation. Under these conditions, a signal of 5,000 to 15,000 cpm for specific binding is consistently obtained. Nonspecific binding is measured in the presence of 10 mM EDTA and was less than 0.4% of total radioactivity.

CTGF Binding to LPR: Preparation of Crude Cell Membranes-BMS2 cells are grown to confluence in roller bottles, and then dissociated with 5 mM EDTA in Dulbecco's PBS lacking calcium and magnesium. Cell pellets are collected by centrifugation and washed. The cells are suspended in hypotonic phosphate buffer (7.5 mM NaP04, pH 7.2) and incubated 10 min on ice. The membranes are disrupted by sonication, and the nuclei were stabilized in buffer containing 10 mM NaPO4, pH 7.2, 10 mM NaCl, and 3 mM MgCl2. The nuclei and whole cells are removed by centrifugation for 5 min at 800 × g, and the supernatant was collected. The supernatant is centrifuged over a cushion of 45% sucrose in Dulbecco's PBS, pH 7.2, for 1 h at 24,000 × g. The membrane fraction located at the sucrose/PBS interface is carefully collected, diluted, and concentrated by centrifugation at 100,000 × g for 15 min. The membrane pellet is resuspended in Dulbecco's PBS, and protein content estimated with the Pierce BCA reagent against an albumin standard (Pierce Chemical Co.). From a preparation of an estimated 109 cells, 10-20 mg of crude membrane protein is frequently obtained.

Preparations of membrane proteins (5 µg each) are solubilized in 0.2% TritonTM X-100, 20% glycerol in Dulbecco's PBS (Buffer A) and the insoluble material is removed by centrifugation (14,000 × g) for 10 min at 4 °C. Fractions from the affinity column are used directly in the solution binding assay (10 µl/fraction). The samples are incubated with 0.2 nM 125I-rhCTGF for 3-4 h. The cross-linker, BS3, is added to a final concentration of 0.5 mM, and the reaction proceeded at room temperature for 15 min. Gel sample buffer is added to each sample, the samples are then heated for 2 min at 100°C, and applied to 5% SDS-PAGE. Following electrophoresis, the gels are dried and analyzed by autoradiography.

### Therapeutic Indications and Methods

It was found by the present applicant that CTGF is expressed in various human tissues.

### Cardiovascular diseases

The human or rat CTGF is highly expressed in cardiovascular related tissues. The expression in the above mentioned tissues demonstrates that the human CTGF or mRNA can be utilized to diagnose of cardiovascular diseases. Additionally the activity of the human CTGF can be modulated to treat cardiovascular diseases.

Heart failure is defined as a pathophysiological state in which an abnormality of cardiac function is responsible for the failure of the heart to pump blood at a rate commensurate with the requirement of the metabolizing tissue. It includes all forms of pumping failures such as high output and low output, acute and chronic, right sided or left sided, systolic or diastolic, independent of the underlying cause.

Myocardial infarction (MI) is generally caused by an abrupt decrease in coronary blood flow that follows a thrombotic occlusion of a coronary artery previously narrowed by arteriosclerosis. MI prophylaxis (primary and secondary prevention) is included as well as the acute treatment of MI and the prevention of complications.

Ischemic diseases are conditions in which the coronary flow is restricted resulting in a perfusion which is inadequate to meet the myocardial requirement for oxygen. This group of diseases includes stable angina, unstable angina and asymptomatic ischemia.

Arrhythmias include all forms of atrial and ventricular tachyarrhythmias, atrial tachycardia, atrial flutter, atrial fibrillation, atrio ventricular reentrant tachycardia, preexitation syndrome, ventricular tachycardia, ventricular flutter, ventricular fibrillation, as well as bradycardic forms of arrhythmias.

Hypertensive vascular diseases include primary as well as all kinds of secondary arterial hypertension, renal, endocrine, neurogenic, others. The genes may be used as drug targets for the treatment of hypertension as well as for the prevention of all complications arising from cardiovascular diseases.

Peripheral vascular diseases are defined as vascular diseases in which arterial and/or venous flow is reduced resulting in an imbalance between blood supply and tissue oxygen demand. It includes chronic peripheral arterial occlusive disease (PAOD), acute arterial thrombosis and embolism, inflammatory vascular disorders, Raynaud's phenomenon and venous disorders.

Atherosclerosis is a cardiovascular disease in which the vessel wall is remodeled, compromising the lumen of the vessel. The atherosclerotic remodeling process involves accumulation of cells, both smooth muscle cells and monocyte/macrophage inflammatory cells, in the intima of the vessel wall. These cells take up lipid, likely from the circulation, to form a mature atherosclerotic lesion. Although the formation of these lesions is a chronic process, occurring over decades of an adult human life, the majority of the morbidity associated with atherosclerosis occurs when a lesion ruptures, releasing thrombogenic debris that rapidly occludes the artery. When such an acute event occurs in the coronary artery, myocardial infarction can ensue, and in the worst case, can result in death.

The formation of the atherosclerotic lesion can be considered to occur in five overlapping stages such as migration, lipid accumulation, recruitment of inflammatory cells, proliferation of vascular smooth muscle cells, and extracellular matrix deposition. Each of these processes can be shown to occur in man and in animal models of atherosclerosis, but the relative contribution of each to the pathology and clinical significance of the lesion is unclear.

Thus, a need exists for therapeutic methods and agents to treat cardiovascular pathologies, such as atherosclerosis and other conditions related to coronary artery disease.

Cardiovascular diseases include but are not limited to disorders of the heart and the vascular system like congestive heart failure, myocardial infarction, ischemic diseases of the heart, all kinds of atrial and ventricular arrhythmias, hypertensive vascular diseases, peripheral vascular diseases, and atherosclerosis.

To high or to low levels of fats in the bloodstream, especially cholesterol, can cause long term problems. The risk to develop atherosclerosis and coronary artery or carotid artery disease (and thus the risk of having a heart attack or stroke) increases with the total cholesterol level increasing. Nevertheless, extremely low cholesterol levels may not be healthy. Examples of disorders of lipid metabolism are hyperlipidemia (abnormally high levels of fats (cholesterol, triglycerides, or both) in the blood, may be caused by family history of hyperlipidemia, obesity, a high fat diet, lack of exercise, moderate to high alcohol consumption, cigarette smoking, poorly controlled diabetes, and an underactive thyroid gland), hereditary hyperlipidemias (type I hyperlipoproteinemia (familial hyperchylomicronemia), type II hyperlipoproteinemia (familial hypercholesterolemia), type III hyperlipoproteinemia, type IV hyperlipoproteinemia, or type V hyperlipoproteinemia), hypolipoproteinemia, lipidoses (caused by abnormalities in the enzymes that metabolize fats), Gaucher's disease, Niemann Pick disease, Fabry's disease, Wolman's disease, cerebrotendinous xanthomatosis, sitosterolemia, Refsum's disease, or Tay Sachs disease.

Kidney disorders may lead to hyper or hypotension. Examples for kidney problems possibly leading to hypertension are renal artery stenosis, pyelonephritis, glomerulonephritis, kidney tumors, polycistic kidney disease, injury to the kidney, or radiation therapy affecting the kidney. Excessive urination may lead to hypotension.

### Applications

The present invention provides CTGF for diagnostic methods for pulmonary hypertension.

### Diagnostics

One example describes CTGF as a biomarker for diagnostic use of pulmonary hypertension. Use of CTGF as a biomarker in diagnostics is based by the comparison of CTGF level in a biological sample from a diseased mammal with the CTGF level in a control sample from a healthy or normal mammal. Does the CTGF level in the diseased mammal differs from the CTGF level in a normal or healthy mammal then the diseased mammal is diagnosed with a disease associated with an altered CTGF level. Furthermore, comparing CTGF levels of a biological sample from a diseased mammal with CTGF levels of control samples from mammals with a CTGF-associated disease already diagnosed with different stages or severity of said disease, allows the diagnose of a CTGF-associated disease of said first diseased mammal and specifying the severity of the CTGF-associated disease. The biological sample is taken from the analogue tissue or body fluid than the control sample.

Normal or standard values for CTGF expression are established by using control samples from healthy or diseased mammalian subjects. A control sample can be obtained by collecting separate or combined body fluids or cell extracts taken from normal mammalian subjects, preferably human, achieving statistical relevant numbers. To obtain the normal or standard CTGF level of the control samples, the samples were subjected to suitable detection methods to detect CTGF polypeptide, polynucleotide or activity. The determination of CTGF level in a mammal subjected to diagnosis is performed analogously by collecting a biological sample from said mammal. Quantities of CTGF levels in biological samples from a mammal subjected to diagnosis are compared with the standard or normal values measured from a control sample. Deviation between standard value (determined from control sample) and subject value (determined from biological sample) establishes the parameters for diagnosing disease. Absolute quantification of CTGF levels measured from biological or control samples may be achieved by comparing those values with values obtained from an experiment in which a known amount of a substantially purified polypeptide is used.

Antibodies which specifically bind CTGF may be used for the diagnosis of disorders characterized by the expression of the biomarker CTGF, or in diagnostic assays to monitor patients being treated achieving guidance for therapy for such a disease. Such a treatment includes medication suitable to treat such a disease, and treatment with CTGF polypeptides or polynucleotides, or agonists, antagonists, and inhibitors of CTGF. Antibodies useful for diagnostic purposes may be prepared in the same manner as those described above for therapeutics. Diagnostic assays for CTGF include methods which utilize the antibody and a label to detect CTGF in human body fluids or in extracts of cells or tissues. The antibodies may be used with or without modification, and may be labeled by covalent or non-covalent joining with a reporter molecule. A wide variety of reporter molecules, several of which are described above, are known in the art and may be used.

A variety of protocols for measuring CTGF, including ELISAs, RIAs, Planar Waveguide technology, and FACS, are known in the art and provide a basis for diagnosing altered or abnormal levels of CTGF expression. Planar Waveguide Technology bioassays are designed to perform multiplexed nucleic acid hybridization assays, immunoaffinity reactions and membrane receptor based assays with high sensitivity and selectivity. The recognition elements specific for the analytes of interest are bound onto the surface in small discrete spots; the transfer of the recognition elements onto the surface is performed using an adequate spotting technology, which requires only minute amounts of recognition elements. Such an arrangement of different recognition elements in an array format allows the simultaneous detection and quantification of hundreds to thousands of different analytes per sample including replicates.

### Reactions on microarrays usually follow a typical scheme:

Recognition elements (e.g. oligonucleotides, cDNAs, or antibodies) are spotted onto the chemically modified planar waveguide surface with typical spot diameters of 100 - 200 µm. The remaining free binding sites on the surface subsequently are being blocked to reduce or eliminate nonspecific binding. In a next step the sample (e.g. fluorescently labeled cDNA or pre-incubated analyte / fluorescently labeled antibody complex) is transferred onto the surface for incubation. The incubation time where a selective recognition and binding between recognition elements and corresponding target molecules (e.g. DNA - DNA hybridization or antigen - antibody interaction) occurs depends on the affinity between the analytes and the immobilized recognition elements. The resulting fluorescing spots can then be detected during readout.

Due to the laterally resolved imaging of the fluorescence signals of the individual spots by a CCD-camera, a large variety of different analytes can be quantified simultaneously, requiring typically sample volumes in the range of 15 µl. Calibration and referencing spots allow for accurate quantification of analytes using just one chip and enable the establishment of dose response and time dependent activity profiles [Pawlak (2002), Duveneck (2002)].

Normal or standard values for CTGF expression are established by using control samples from healthy or diseased mammalian subjects. A control sample can be obtained by collecting separate or combined body fluids or cell extracts taken from normal mammalian subjects, preferably human, achieving statistical relevant numbers. To obtain normal or standard values the control samples are combined with an antibody to CTGF under conditions suitable for complex formation. The amount of standard complex formation may be quantified by various methods, preferably by photometric means. The determination of CTGF level in a mammal subjected to diagnosis is performed analogously by collecting a biological sample from said mammal, combining said sample with an antibody to CTGF and determination of complex formation. Quantities of CTGF expressed in biological samples from a mammal subjected to diagnosis are compared with the standard or normal values measured from a control sample. Deviation between standard value (determined from control sample) and subject value (determined from biological sample) establishes the parameters for diagnosing disease. Absolute quantification of CTGF levels measured from biological or control samples may be achieved by comparing those values with values obtained from an experiment in which a known amount of a substantially purified polypeptide is used.

In another example, the polynucleotides encoding CTGF may be used for diagnostic purposes. The polynucleotides which may be used include oligonucleotide sequences, complementary RNA and DNA molecules, and PNAs. The polynucleotides may be used to detect and quantified gene expression in control and biological samples in which expression of the biomarker CTGF may be correlated with disease. The diagnostic assay may be used to distinguish between absence, presence, and excess expression of CTGF, and to monitor regulation of CTGF levels during therapeutic intervention.

Polynucleotide sequences encoding CTGF may be used for the diagnosis of cardiovascular diseases and hematological diseases associated with expression of CTGF. The polynucleotide sequences encoding CTGF may be used in Southern, Northern, or dot-blot analysis, or other membrane-based technologies; in PCR technologies; in dipstick, pin, and ELISA assays; bDNA (branched DNA technology) and Planar Waveguide Technology; and in microarrays utilizing a biological sample from diseased mammals to detect altered CTGF expression. Such qualitative or quantitative methods are well known in the art.

In a particular aspect, the nucleotide sequences encoding CTGF may be useful in assays that detect the presence of associated disorders, particularly those mentioned above. The nucleotide sequences encoding CTGF may be labeled by standard methods and added to a biological sample from diseased mammals under conditions suitable for the formation of hybridization complexes. After a suitable incubation period, the sample is washed and the signal is quantified and compared with a standard value. If the amount of signal in the patient sample is altered from that of a comparable control sample, the nucleotide sequences have hybridized with nucleotide sequences in the sample, and the presence of altered levels of nucleotide sequences encoding CTGF in the sample indicates the presence of the associated disorder. Such assays may also be used to evaluate the efficacy of a particular therapeutic treatment regimen in animal studies, in clinical trials, or in monitoring the treatment of an individual patient.

In order to provide a basis for the diagnosis of cardiovascular diseases and hematological diseases associated with expression of CTGF, a normal or standard profile for expression is established. This may be accomplished by combining body fluids or cell extracts taken from normal subjects, either animal or human, with a sequence, or a fragment thereof, encoding CTGF, under conditions suitable for hybridization or amplification. Quantification of CTGF levels measured from biological or control samples may be achieved by comparing those values with values obtained from an experiment in which a known amount of a substantially purified polynucleotide is used. Standard values obtained from normal samples may be compared with values obtained from samples from patients who are symptomatic for a disorder. Deviation from standard values is used to establish the presence of a disorder.

### Biomarker

### Use of CTGF as a biomarker

One of ordinary skill in the art knows several methods and devices for the detection and analysis of the markers of the instant invention. With regard to polypeptides or proteins in patient test samples, immunoassay devices and methods are often used. These devices and methods can utilize labelled molecules in various sandwich, competitive, or non-competitive assay formats, to generate a signal that is related to the presence or amount of an analyte of interest. Additionally, certain methods and devices, such as biosensors and optical immunoassays, may be employed to determine the presence or amount of analytes without the need for a labelled molecule.

Preferably the markers are analyzed using an immunoassay, although other methods are well known to those skilled in the art (for example, the measurement of marker RNA levels). The presence or amount of a marker is generally determined using antibodies specific for each marker and detecting specific binding. Any suitable immunoassay may be utilized, for example, enzyme-linked immunoassays (ELISA), radioimmunoassay (RIAs), competitive binding assays, planar waveguide technology, and the like. Specific immunological binding of the antibody to the marker can be detected directly or indirectly. Direct labels include fluorescent or luminescent tags, metals, dyes, radionuclides, and the like, attached to the antibody. Indirect labels include various enzymes well known in the art, such as alkaline phosphatase, horseradish peroxidase and the like. For an example of how this procedure is carried out on a machine, one can use the RAMP Biomedical device, called the Clinical Reader sup™, which uses the fluorescent tag method, though the skilled artisan will know of many different machines and manual protocols to perform the same assay. Diluted whole blood is applied to the sample well. The red blood cells are retained in the sample pad, and the separated plasma migrates along the strip. Fluorescent dyed latex particles bind to the analyte and are immobilized at the detection zone. Additional particles are immobilized at the internal control zone. The fluorescence of the detection and internal control zones are measured on the RAMP Clinical Reader sup™, and the ratio between these values is calculated. This ratio is used to determine the analyte concentration by interpolation from a lot-specific standard curve supplied by the manufacturer in each test kit for each assay.

The use of immobilized antibodies specific for the markers is also contemplated by the present invention and is well known by one of ordinary skill in the art. The antibodies could be immobilized onto a variety of solid supports, such as magnetic or chromatographic matrix particles, the surface of an assay place (such as microtiter wells), pieces of a solid substrate material (such as plastic, nylon, paper), and the like. An assay strip could be prepared by coating the antibody or a plurality of antibodies in an array on solid support. This strip could then be dipped into the test sample and then processed quickly through washes and detection steps to generate a measurable signal, such as a coloured spot.

The analysis of a plurality of markers may be carried out separately or simultaneously with one test sample. Several markers may be combined into one test for efficient processing of a multiple of samples. In addition, one skilled in the art would recognize the value of testing multiple samples (for example, at successive time points) from the same individual. Such testing of serial samples will allow the identification of changes in marker levels over time. Increases or decreases in marker levels, as well as the absence of change in marker levels, would provide useful information about the disease status that includes, but is not limited to identifying the approximate time from onset of the event, the presence and amount of salvagable tissue, the appropriateness of drug therapies, the effectiveness of various therapies, identification of the severity of the event, identification of the disease severity, and identification of the patient's outcome, including risk of future events.

An assay consisting of a combination of the markers referenced in the instant invention may be constructed to provide relevant information related to differential diagnosis. Such a panel may be constucted using 1, 2, 3, 4, 5, 6, 7, 8, 9, 10, 15, 20, or more or individual markers. The analysis of a single marker or subsets of markers comprising a larger panel of markers could be carried out methods described within the instant invention to optimize clinical sensitivity or specificity in various clinical settings.

The analysis of markers could be carried out in a variety of physical formats as well. For example, the use of microtiter plates or automation could be used to facilitate the processing of large numbers of test samples. Alternatively, single sample formats could be developed to facilitate immediate treatment and diagnosis in a timely fashion, for example, in ambulatory transport or emergency room settings. Particularly useful physical formats comprise surfaces having a plurality of discrete, addressable locations for the detection of a plurality of different analytes. Such formats include protein microarrays, or "protein chips" and capillary devices.

Cardiac markers serve an important role in the early detection and monitoring of cardiovascular disease. Markers of disease are typically substances found in a bodily sample that can be easily measured. The measured amount can correlate to underlying disease pathophysiology, presence or absence of a current or imminent cardiac event, probability of a cardiac event in the future. In patients receiving treatment for their condition the measured amount will also correlate with responsiveness to therapy. Markers can include elevated levels of blood pressure, cholesterol, blood sugar, homocysteine and C- reactive protein (CRP). However, current markers, even in combination with other measurements or risk factors, do not adequately identify patients at risk, accurately detect events (i.e., heart attacks), or correlate with therapy. For example, half of patients do not have elevated serum cholesterol or other traditional risk factors.

Use of markers in diagnosis of cardiac conditions is described in, for example, Alpert et al. (2000); Newby et al. (2001); de Lemos et al. (2002); Boersma et al. (2002); Christenson et al. (2001).

### Cardiovascular biomarker

### BNP (as an example for cardiovascular biomarkers)

B-type natriuretic peptide (BNP), also called brain- type natriuretic peptide is a 32 amino acid, 4 kDa peptide that is involved in the natriuresis system to regulate blood pressure and fluid balance. The precursor to BNP is synthesized as a 108-amino acid molecule, referred to as "pre pro BNP," that is proteolytically processed into a 76-amino acid N-terminal peptide (amino acids 1-76), referred to as "NT pro BNP" and the 32-amino acid mature hormone, referred to as BNP or BNP 32 (amino acids 77-108). It has been suggested that each of these species-NT pro- BNP, BNP-32, and the pre pro BNP--can circulate in human plasma. The 2 forms, pre pro BNP and NT pro BNP, and peptides which are derived from BNP, pre pro BNP and NT pro BNP and which are present in the blood as a result of proteolyses of BNP, NT pro BNP and pre pro BNP, are collectively described as markers related to or associated with BNP. Proteolytic degradation of BNP and of peptides related to BNP have also been described in the literature and these proteolytic fragments are also encompassed it the term "BNP related peptides". BNP and BNP-related peptides are predominantly found in the secretory granules of the cardiac ventricles, and are released from the heart in response to both ventricular volume expansion and pressure overload. Elevations of BNP are associated with raised atrial and pulmonary wedge pressures, reduced ventricular systolic and diastolic function, left ventricular hypertrophy, and myocardial infarction [Sagnella, (1998)]. Furthermore, there are numerous reports of elevated BNP concentration associated with congestive heart failure and renal failure. While BNP and BNP-related peptides are likely not specific for ACS, they may be sensitive markers of ACS because they may indicate not only cellular damage due to ischemia, but also a perturbation of the natriuretic system associated with ACS. The term "BNP" as used herein refers to the mature 32-amino acid BNP molecule itself. As the skilled artisan will recognize, however, other markers related to BNP may also serve as diagnostic or prognostic indicators in patients with ACS. For example, BNP is synthesized as a 108-amino acid pre pro-BNP molecule that is proteolytically processed into a 76-amino acid "NT pro BNP" and the 32- amino acid BNP molecule. Because of its relationship to BNP, the concentration of NT pro-BNP molecule can also provide diagnostic or prognostic information in patients. The phrase "marker related to BNP or BNP related peptide" refers to any polypeptide that originates from the pre pro-BNP molecule, other than the 32-amino acid BNP molecule itself. Thus, a marker related to or associated with BNP includes the NT pro-BNP molecule, the pro domain, a fragment of BNP that is smaller than the entire 32-amino acid sequence, a fragment of pre pro-BNP other than BNP, and a fragment of the pro domain.

### Biomarker classes

CTGF could be used as a biomarker for cardiovascular diseases and hematological diseases in different classes :
Disease Biomarker: a biomarker that relates to a clinical outcome or measure of disease.
Efficacy Biomarker: a biomarker that reflects beneficial effect of a given treatment.
Staging Biomarker: a biomarker that distinguishes between different stages of a chronic disorder.
Surrogate Biomarker: a biomarker that is regarded as a valid substitute for a clinical outcomes measure.
Toxicity Biomarker: a biomarker that reports a toxicological effect of a drug on an *in vitro* or *in vivo* system.
Mechanism Biomarker: a biomarker that reports a downstream effect of a drug.
Target Biomarker: a biomarker that reports interaction of the drug with its target.

Disclosed is a method of use of CTGF as a biomarker for a disease comprising :
(a) obtaining a biological sample from a mammal,
(b) measuring the level of CTGF in the biological sample,
(c) obtaining a control sample from a mammal,
(d) measuring the level of CTGF in the control sample,
(e) comparing the level of CTGF in the biological sample with the level of CTGF in a control sample, and
(f) diagnosing a disease based upon the CTGF level of the biological sample in comparison to the control sample.

The biological sample in step (a) of the methods is in a preferred example a biological sample comprised in a group of samples consisting of a blood sample, a plasma sample, a serum sample, a tissue sample, a oral mucosa sample, a saliva sample, an interstitial fluid sample or an urine sample. The blood sample is for example a whole blood sample, a fractionated blood sample, a platelet sample, a neutrophil sample, a leukocyte sample, a white blood cell sample, a monocyte sample, a red blood cell sample, a granulocyte sample, and a erythrocyte sample. A tissue sample is for example a sample collected from muscle, adipose, heart or skin.

In one disclosure CTGF is used as a biomarker diagnosing a disease which is associated with altered CTGF levels. In another disclosure CTGF is used as a biomarker for identifying an individual risk for developing a disease, or for predicting an adverse outcome in a patient diagnosed with a disease,

Use of CTGF as a disease biomarker in diagnostics is based by the comparison of CTGF level in a biological sample from a diseased mammal with the CTGF level in a control sample from a healthy or normal mammal or a group of healthy or normal mammals. Does the CTGF level in the diseased mammal differs from the CTGF level in a normal or healthy mammal then the diseased mammal is diagnosed with a disease associated with altered CTGF level.

Furthermore, using CTGF as a staging biomarker, the CTGF levels of a diseased mammal are compared with CTGF levels of a mammal with a CTGF-associated disease already diagnosed with different stages or severity of said disease, allows the diagnose of said first diseased mammal specifying the severity of the CTGF-associated disease.

A control sample can be a sample taken from a mammal. A control sample can be a previously taken sample from a mammal, as a CTGF level in a control sample can be a predetermined level of CTGF measured in a previously taken sample. The level of CTGF in a control sample or in a biological sample can be determined for example as a relative value and as an absolute value. A previously measured CTGF level from a control sample can be for example stored in a database, in an internet publication, in an electronically accessible form, in a publication. Comparing the level of CTGF of a biological sample to a control sample may be comparing relative values or absolute quantified values.

Another embodiment is a method of use of CTGF as a disease , efficacy or surrogate endpoint biomarker for a therapy of pulmonary hypertension according to claim 1.

Use of CTGF as a disease, efficacy or surrogate endpoint biomarker in diagnostics is based by the comparison of CTGF level in a biological sample from a diseased mammal before treatment (the baseline sample level) with the CTGF level in subsequent samples from said mammal receiving a treatment for the disease. Does the CTGF level in the baseline sample differs from the CTGF level in the subsequent samples then the therapy can be considered as successful. Does the CTGF level in the baseline sample does not differ or differs only slightly from the CTGF level in the subsequent samples then the therapy can be considered as not successful. If the therapy is considered not successful increased dosages of the same therapy, repeat of the same therapy or an alternative treatment which is different from the first therapy can be considered.

The biological sample in step (a) of the methods is collected from heart.

In a preferred embodiment the level of CTGF is determined by determining the level of CTGF polynucleotide.

In another preferred embodiment the level of CTGF is determined by determining the level of CTGF polypeptide.

In a further preferred embodiment the level of CTGF is determined by determining the level of CTGF activity.

In one disclosure the disease associated with CTGF is comprised in a group of diseases consisting of cardiovascular diseases, hematological diseases, neurological diseases, respiratory diseases, gastroenterological diseases, and urological diseases. In a more preferred example the cardiovascular disease associated with CTGF is comprised in a group of diseases consisting of congestive heart failure, pulmonary hypertension, left ventricular dysfunction, and right ventricular dysfunction, myocardial infarction, coronary occlusion, disease, ischemic heart disease, cardiac hypertrophy disorder, cardiac fibrosis disorders.

In a preferred embodiment of the invention the mammal is a human.

In a preferred embodiment of the invention the level of CTGF of the biological sample is elevated compared to the control sample.

Another embodiment of the present invention prefers the use of CTGF in combination with the use of one or more biomarkers, more preferably with biomarkers used in diagnosing CTGF-associated diseases.

In a preferred embodiment of the invention the use of CTGF is combined with the use of one or more biomarkers which are comprised in a group of biomarkers consisting of CRTAC, PRSS23, FN1, LTBP2, TGFB2, NPR3, BNP, ANP, Troponin, CRP, Myoglobin, CK-MB and metabolites.

In a further preferred embodiment the use of CTGF is combined with the use of one or more clinical biomarkers which are comprised in a group of biomarkers consisting of blood pressure, heart rate, pulmonary artery pressure, or systemic vascular resistance.

In a further preferred embodiment the use of CTGF is combined with the use of one or more diagnostic imaging methods which are comprised in a group of methods consisting of PET (Positron Emission Tomography), CT (Computed Tomography), ultrasonic, SPECT (Single Photon Emission Computed Tomography), Echocardiography, or Impedance Cardiography.

In a further preferred embodiment the use of CTGF is combined with the use of one or more diagnostic imaging methods which are comprised in a group of methods consisting of PET (Positron Emission Tomography), CT (Computed Tomography), ultrasonic, SPECT (Single Photon Emission Computed Tomography), Echocardiography, Impedance Cardiography, blood pressure, heart rate, pulmonary artery pressure, systemic vascular resistance, CRTAC, PRSS23, FN1, LTBP2, TGFB2, NPR3, BNP, ANP, Troponin, CRP, Myoglobin, CK-MB, and metabolites.

Further disclosed is a kit for identifying an individual risk for developing a disease, for predicting a disease or an adverse outcome in a patient diagnosed with a disease, or for guiding a therapy in a patient with a disease, the kit comprising one ore more antibodies which specifically binds CTGF, detection means, one or more containers for collecting and or holding the biological sample, and an instruction for its use.

Disclosed is a kit for identifying an individual risk for developing a disease, for predicting a disease or an adverse outcome in a patient diagnosed with a disease, or for guiding a therapy in a patient with a disease, the kit comprising one or more probes or primers for detecting CTGF mRNA, detection means, one or more containers for collecting and or holding the biological sample, and an instruction for its use.

Disclosed is a kit for identifying an individual risk for developing a disease, for predicting a disease or an adverse outcome in a patient diagnosed with a disease, or for guiding a therapy in a patient with a disease, the kit comprising one or more substrates for detecting CTGF activity, detection means, one or more containers for collecting and or holding the biological sample, and an instruction for its use.

### Determination of a Therapeutically Effective Dose

The determination of a therapeutically effective dose is well within the capability of those skilled in the art. A therapeutically effective dose refers to that amount of active ingredient which increases or decreases CTGF activity relative to CTGF activity which occurs in the absence of the therapeutically effective dose. For any compound, the therapeutically effective dose can be estimated initially either in cell culture assays or in animal models, usually mice, rabbits, dogs, or pigs. The animal model also can be used to determine the appropriate concentration range and route of administration. Such information can then be used to determine useful doses and routes for administration in humans.

Therapeutic efficacy and toxicity, e.g., ED₅₀ (the dose therapeutically effective in 50% of the population) and LD₅₀ (the dose lethal to 50% of the population), can be determined by standard pharmaceutical procedures in cell cultures or experimental animals. The dose ratio of toxic to therapeutic effects is the therapeutic index, and it can be expressed as the ratio, LD₅₀/ED₅₀. Pharmaceutical compositions which exhibit large therapeutic indices are preferred. The data obtained from cell culture assays and animal studies is used in formulating a range of dosage for human use. The dosage contained in such compositions is preferably within a range of circulating concentrations that include the ED₅₀ with little or no toxicity. The dosage varies within this range depending upon the dosage form employed, sensitivity of the patient, and the route of administration. The exact dosage will be determined by the practitioner, in light of factors related to the subject that requires treatment. Dosage and administration are adjusted to provide sufficient levels of the active ingredient or to maintain the desired effect. Factors which can be taken into account include the severity of the disease state, general health of the subject, age, weight, and gender of the subject, diet, time and frequency of administration, drug combination(s), reaction sensitivities, and tolerance/response to therapy. Long-acting pharmaceutical compositions can be administered every 3 to 4 days, every week, or once every two weeks depending on the half-life and clearance rate of the particular formulation.

Normal dosage amounts can vary from 0.1 micrograms to 100,000 micrograms, up to a total dose of about 1 g, depending upon the route of administration. Guidance as to particular dosages and methods of delivery is provided in the literature and generally available to practitioners in the art. Those skilled in the art will employ different formulations for nucleotides than for proteins or their inhibitors. Similarly, delivery of polynucleotides or polypeptides will be specific to particular cells, conditions, locations, etc. If the reagent is a single-chain antibody, polynucleotides encoding the antibody can be constructed and introduced into a cell either *ex vivo* or *in vivo* using well-established techniques including, but not limited to, transferrin-polycation-mediated DNA transfer, transfection with naked or encapsulated nucleic acids, liposome-mediated cellular fusion, intracellular transportation of DNA-coated latex beads, protoplast fusion, viral infection, electroporation, "gene gun", and DEAE- or calcium phosphate-mediated transfection.

If the expression product is mRNA, the reagent is preferably an antisense oligonucleotide or a ribozyme. Polynucleotides which express antisense oligonucleotides or ribozymes can be introduced into cells by a variety of methods, as described above. Preferably, a reagent reduces expression of CTGF gene or the activity of CTGF by at least about 1 0, preferably about 50, more preferably about 75, 90, or 100% relative to the absence of the reagent. The effectiveness of the mechanism chosen to decrease the level of expression of CTGF gene or the activity of CTGF can be assessed using methods well known in the art, such as hybridization of nucleotide probes to CTGF-specific mRNA, quantitative RT-PCR, immunologic detection of CTGF, or measurement of CTGF activity.

In any of the embodiments described above, any of the pharmaceutical compositions of the invention can be administered in combination with other appropriate therapeutic agents. Selection of the appropriate agents for use in combination therapy can be made by one of ordinary skill in the art, according to conventional pharmaceutical principles. The combination of therapeutic agents can act synergistically to effect the treatment or prevention of the various disorders described above. Using this approach, one may be able to achieve therapeutic efficacy with lower dosages of each agent, thus reducing the potential for adverse side effects. Any of the therapeutic methods described above can be applied to any subject in need of such therapy, including, for example, mammals such as dogs, cats, cows, horses, rabbits, monkeys, and most preferably, humans.

Nucleic acid molecules disclosed in the invention are those nucleic acid molecules which are contained in a group of nucleic acid molecules consisting of (i) nucleic acid molecules encoding a polypeptide comprising the amino acid sequence of SEQ ID NO: 3 or SEQ ID NO: 4, (ii) nucleic acid molecules comprising the sequence of SEQ ID NO: 1 or SEQ ID NO: 2, (iii) nucleic acid molecules having the sequence of SEQ ID NO: I or SEQ ID NO: 2 , (iv) nucleic acid molecules the complementary strand of which hybridizes under stringent conditions to a nucleic acid molecule of (i), (ii), or (iii), (v) nucleic acid molecules the sequence of which differs from the sequence of a nucleic acid molecule of (iii) due to the degeneracy of the genetic code, (vi) nucleic acid molecules which have a sequence identity of at least 80%, 85%, 90%, 95%, 98% or 99%; and (vii) wherein the polypeptide encoded by said nucleic acid molecules of (i)-(vi) have CTGF activity.

Polypeptides disclosed are those polypeptides which are contained in a group of polypeptides consisting of (i) polypeptides having the sequence of SEQ ID NO: 3 or 4, (ii) polypeptides comprising the sequence of SEQ ID NO: 3 or 4, (iii) polypeptides encoded by nucleic acid molecules of the invention and (iv) polypeptides which show at least 99%, 98%, 95%, 90%, or 80% identity with a polypeptide of (i), (ii), or (iii).

Another disclosure is a method of diagnosing a disease comprised in a group of diseases consisting of cardiovascular diseases and hematological diseases in a mammal comprising the steps of (i) determining the amount of a CTGF polynucleotide in a sample taken from said mammal, (ii) determining the amount of CTGF polynucleotide in healthy and/or diseased mammal. A disease is diagnosed, e.g., if there is a substantial similarity in the amount of CTGF polynucleotide in said test mammal as compared to a diseased mammal.

The uses, methods or compositions of the invention are useful for each single disease comprised in a group of diseases consisting of cardiovascular diseases and hematological diseases.

The examples below are provided to illustrate the subject invention. These examples are provided by way of illustration and are not included for the purpose of limiting the invention.

### Examples

### Example 1: Search for homologous sequences in public sequence data bases

The degree of homology can readily be calculated by known methods. Preferred methods to determine homology are designed to give the largest match between the sequences tested. Methods to determine homology are codified in publicly available computer programs such as BestFit, BLASTP, BLASTN, and FASTA. The BLAST programs are publicly available from NCBI and other sources in the internet.

For CTGF the following hits to known sequences were identified by using the BLAST algorithm [Altschul SF, Madden TL, Schaffer AA, Zhang J, Zhang Z, Miller W, Lipman DJ; Nucleic Acids Res 1997 Sep 1; 25(17): 3389-402] and the following set of parameters: matrix = BLOSUM62 and low complexity filter. The following databases were searched: NCBI (non-redundant database) and DERWENT patent database (Geneseq).

The following hits were found:
>dbj|DD288037.1| LYMPHATIC ENDOTHELIAL GENES, Length = 7017, Score = 1.391e+04 bits (7017), Expect = 0.0, Identities = 7017/7017 (100%)
>gb|S82451.1| latent transforming growth factor-beta-binding protein-2 [human, fibroblast cell line CC102, mRNA, 7017 nt], Length = 7017, Score = 1.391e+04 bits (7017), Expect = 0.0. Identities = 7017/7017 (100%)
>ref|NM_000428.2| Homo sapiens latent transforming growth factor beta binding protein 2 (CTGF), mRNA, Length = 8568, Score = 1.384e+04 bits (6982), Expect = 0.0, Identities = 6982/6982 (100%)
>dbj|DD018049.1| A marker of heart failure and its use Length = 8657, Score = 1.376e+04 bits (6940), Expect = 0.0, Identities = 6967/697.2 (99%), Gaps = 3/6972 (0%)
>emb|CQ874661.1| Sequence 21 from Patent WO2004075835, Length = 7000, Score = 1.369e+04 bits (6906), Expect = 0.0, Identities = 6954/6966 (99%), Gaps = 3/6966 (0%)
>emb|Z37976.1|HSCTGFMR H.sapiens mRNA for latent transforming growth factor-beta binding protein (LTBP-2). Length = 7000, Score = 1.369e+04 bits (6906), Expect = 0.0, Identities = 6954/6966 (99%), Gaps = 3/6966 (0%)
>gb|BC078659.1| Homo sapiens latent transforming growth factor beta binding protein 2, mRNA (cDNA clone MGC:87426 IMAGE:30343778), complete cds, Length = 6901, Score = 1.361e+04 bits (6867), Expect = 0.0, Identities = 6897/6905 (99%), Gaps = 4/6905 (0%)
>dbj|AB209865.1| Homo sapiens mRNA for latent transforming growth factor beta binding protein 2 variant protein, Length = 7803, Score = 1.232e+04 bits (6216), Expect = 0.0, Identities = 6228/6232 (99%)

### Example 2: Antisense Analysis

Knowledge of the correct, complete cDNA sequence coding for CTGF enables its use as a tool for antisense technology in the investigation of gene function. Oligonucleotides, cDNA or genomic fragments comprising the antisense strand of a polynucleotide coding for CTGF are used either in vitro or in vivo to inhibit translation of the mRNA. Such technology is now well known in the art, and antisense molecules can be designed at various locations along the nucleotide sequences. By treatment of cells or whole test animals with such antisense sequences, the gene of interest is effectively turned off. Frequently, the function of the gene is ascertained by observing behavior at the intracellular, cellular, tissue or organismal level (e.g., lethality, loss of differentiated function, changes in morphology, etc.).

In addition to using sequences constructed to interrupt transcription of a particular open reading frame, modifications of gene expression is obtained by designing antisense sequences to intron regions, promoter/enhancer elements, or even to trans-acting regulatory genes.

### Example 3: Expression of CTGF

Expression of CTGF is accomplished by subcloning the cDNAs into appropriate expression vectors and transfecting the vectors into expression hosts such as, e.g., *E. coli.* In a particular case, the vector is engineered such that it contains a promoter for β-galactosidase, upstream of the cloning site, followed by sequence containing the amino-terminal Methionine and the subsequent seven residues of β-galactosidase. Immediately following these eight residues is an engineered bacteriophage promoter useful for artificial priming and transcription and for providing a number of unique endonuclease restriction sites for cloning.

Induction of the isolated, transfected bacterial strain with Isopropyl-β-D-thiogalactopyranoside (IPTG) using standard methods produces a fusion protein corresponding to the first seven residues of β-galactosidase, about 15 residues of "linker", and the peptide encoded within the cDNA. Since DNA clone inserts are generated by an essentially random process, there is probability of 33% that the included cDNA will lie in the correct reading frame for proper translation. If the cDNA is not in the proper reading frame, it is obtained by deletion or insertion of the appropriate number of bases using well known methods including in vitro mutagenesis, digestion with exonuclease III or mung bean nuclease, or the inclusion of an oligonucleotide linker of appropriate length.

The CTGF cDNA is shuttled into other vectors known to be useful for expression of proteins in specific hosts. Oligonucleotide primers containing cloning sites as well as a segment of DNA (about 25 bases) sufficient to hybridize to stretches at both ends of the target cDNA is synthesized chemically by standard methods. These primers are then used to amplify the desired gene segment by PCR. The resulting gene segment is digested with appropriate restriction enzymes under standard conditions and isolated by gel electrophoresis. Alternately, similar gene segments are produced by digestion of the cDNA with appropriate restriction enzymes. Using appropriate primers, segments of coding sequence from more than one gene are ligated together and cloned in appropriate vectors. It is possible to optimize expression by construction of such chimeric sequences.

Suitable expression hosts for such chimeric molecules include, but are not limited to, mammalian cells such as Chinese Hamster Ovary (CHO) and human 293 cells., insect cells such as Sf9 cells, yeast cells such as *Saccharomyces cerevisiae* and bacterial cells such as *E*. *coli.* For each of these cell systems, a useful expression vector also includes an origin of replication to allow propagation in bacteria, and a selectable marker such as the β-lactamase antibiotic resistance gene to allow plasmid selection in bacteria. In addition, the vector may include a second selectable marker such as the neomycin phosphotransferase gene to allow selection in transfected eukaryotic host cells. Vectors for use in eukaryotic expression hosts require RNA processing elements such as 3' polyadenylation sequences if such are not part of the cDNA of interest.

Additionally, the vector contains promoters or enhancers which increase gene expression. Such promoters are host specific and include MMTV, SV40, and metallothionine promoters for CHO cells; trp, lac, tac and T7 promoters for bacterial hosts; and alpha factor, alcohol oxidase and PGH promoters for yeast. Transcription enhancers, such as the rous sarcoma virus enhancer, are used in mammalian host cells. Once homogeneous cultures of recombinant cells are obtained through standard culture methods, large quantities of recombinantly produced CTGF are recovered from the conditioned medium and analyzed using chromatographic methods known in the art. For example, CTGF can be cloned into the expression vector pcDNA3, as exemplified herein. This product can be used to transform, for example, HEK293 or COS by methodology standard in the art. Specifically, for example, using Lipofectamine (Gibco BRL catolog no. 18324-020) mediated gene transfer.

### Example 4: Isolation of Recombinant CTGF

CTGF is expressed as a chimeric protein with one or more additional polypeptide domains added to facilitate protein purification. Such purification facilitating domains include, but are not limited to, metal chelating peptides such as histidine-tryptophan modules that allow purification on immobilized metals [Appa Rao, (1997)] and the domain utilized in the FLAGS extension/affinity purification system (Immunex Corp., Seattle, Washington). The inclusion of a cleavable linker sequence such as Factor Xa or enterokinase (Invitrogen, Groningen, The Netherlands) between the purification domain and the CTGF sequence is useful to facilitate expression of CTGF.

The following example provides a method for purifying CTGF.

CTGF is generated using the baculovirus expression system BAC-TO-BAC (GIBCO BRL) based on *Autographa californica* nuclear polyhedrosis virus (AcNPV) infection of *Spodoptera frugiperda* insect cells (Sf9 cells).

cDNA encoding proteins cloned into either the donor plasmid pFASTBAC1 or pFASTBAC-HT which contain a mini-Tn7 transposition element. The recombinant plasmid is transformed into DH10BAC competent cells which contain the parent bacmid bMON14272 (AcNPV infectious DNA) and a helper plasmid. The mini-Tn7 element on the pFASTBAC donor can transpose to the attTn7 attachment site on the bacmid thus introducing the gene into the viral genome. Colonies containing recombinant bacmids are identified by disruption of the *lac*Z gene. The bacmid construct can then be isolated and infected into insect cells (Sf9 cells) resulting in the production of infectious recombinant baculovirus particles and expression of either unfused recombinant enzyme (pFastbac1) or CTGF-His fusion protein (pFastbacHT).

Cells are harvested and extracts prepared 24, 48 and 72 hours after transfection. Expression of CTGF is confirmed by coomassie staining after sodium dodecyl sulphate-polyacrylamide gel electrophoresis (SDS-PAGE) and western blotting onto a PVDF membrane of an unstained SDS-' PAGE. The protein-His fusion protein is detected due to the interaction between the Ni-NTA HRP conjugate and the His-tag which is fused to CTGF.

### Example 5: Production of CTGF Specific Antibodies

Two approaches are utilized to raise antibodies to CTGF, and each approach is useful for generating either polyclonal or monoclonal antibodies. In one approach, denatured protein from reverse phase HPLC separation is obtained in quantities up to 75 mg. This denatured protein is used to immunize mice or rabbits using standard protocols; about 100 µg are adequate for immunization of a mouse, while up to 1 mg might be used to immunize a rabbit. For identifying mouse hybridomas, the denatured. protein is radioiodinated and used to screen potential murine B-cell hybridomas for those which produce antibody. This procedure requires only small quantities of protein, such that 20 mg is sufficient for labeling and screening of several thousand clones.

In the second approach, the amino acid sequence of an appropriate CTGF domain, as deduced from translation of the cDNA, is analyzed to determine regions of high antigenicity. Oligopeptides comprising appropriate hydrophilic regions are synthesized and used in suitable immunization protocols to raise antibodies. The optimal amino acid sequences for immunization are usually at the C-terminus, the N-terminus and those intervening, hydrophilic regions of the polypeptide which are likely to be exposed to the external environment when the protein is in its natural conformation.

Typically, selected peptides, about 15 residues in length, are synthesized using an Applied Biosystems Peptide Synthesizer Model 431 A using fmoc-chemistry and coupled to keyhole limpet hemocyanin (KLH; Sigma, St. Louis, MO) by reaction with M-maleimidobenzoyl-N-hydroxysuccinimide ester, MBS. If necessary, a cysteine is introduced at the N-terminus of the peptide to permit coupling to KLH. Rabbits are immunized with the peptide-KLH complex in complete Freund's adjuvant. The resulting antisera are tested for antipeptide activity by binding the peptide to plastic, blocking with 1% bovine serum albumin, reacting with antisera, washing and reacting with labeled (radioactive or fluorescent), affinity purified, specific goat anti-rabbit IgG.

Hybridomas are prepared and screened using standard techniques. Hybridomas of interest are detected by screening with labeled CTGF to identify those fusions producing the monoclonal antibody with the desired specificity. In a typical protocol, wells of plates (FAST; Becton-Dickinson, Palo Alto, CA) are coated during incubation with affinity purified, specific rabbit antimouse (or suitable antispecies 1 g) antibodies at 10 mg/ml. The coated wells are blocked with 1% bovine serum albumin, (BSA), washed and incubated with supernatants from hybridomas. After washing the wells are incubated with labeled CTGF at 1 mg/ml. Supernatants with specific antibodies bind more labeled CTGF than is detectable in the background. Then clones producing specific antibodies are expanded and subjected to two cycles of cloning at limiting dilution. Cloned hybridomas are injected into pristane-treated mice to produce ascites, and monoclonal antibody is purified from mouse ascitic fluid by affinity chromatography on Protein A. Monoclonal antibodies with affinities of at least

10⁸ M⁻¹, preferably 10⁹ to 10¹⁰ M⁻¹ or stronger, are typically made by standard procedures.

### Example 6: Diagnostic Test Using CTGF Specific Antibodies

Particular CTGF antibodies are useful for investigating signal transduction and the diagnosis of infectious or hereditary conditions which are characterized by differences in the amount or distribution of CTGF or downstream products of an active signaling cascade.

Diagnostic tests for CTGF include methods utilizing antibody and a label to detect CTGF in human body fluids, membranes, cells, tissues or extracts of such. The polypeptides and antibodies of the present invention are used with or without modification. Frequently, the polypeptides and antibodies are labeled by joining them, either covalently or noncovalently, with a substance which provides for a detectable signal. A wide variety of labels and conjugation techniques are known and have been reported extensively in both the scientific and patent literature. Suitable labels include radionuclides, enzymes, substrates, cofactors, inhibitors, fluorescent agents, chemiluminescent agents, chromogenic agents, magnetic particles and the like.

A variety of protocols for measuring soluble or membrane-bound CTGF, using either polyclonal or monoclonal antibodies specific for the protein, are known in the art. Examples include enzyme-linked immunosorbent assay (ELISA), radioimmunoassay (RIA) and fluorescent activated cell sorting (FACS). A two-site monoclonal-based immunoassay utilizing monoclonal antibodies reactive to two non-interfering epitopes on CTGF is preferred, but a competitive binding assay may be employed.

### Example 7: Purification of Native CTGF Using Specific Antibodies

Native or recombinant CTGF is purified by immunoaffinity chromatography using antibodies specific for CTGF. In general, an immunoaffinity column is constructed by covalently coupling the anti-TRH antibody to an activated chromatographic resin.

Polyclonal immunoglobulins are prepared from immune sera either by precipitation with ammonium sulfate or by purification on immobilized Protein A (Pharmacia LKB Biotechnology, Piscataway N.J.). Likewise, monoclonal antibodies are prepared from mouse ascites fluid by ammonium sulfate precipitation or chromatography on immobilized Protein A. Partially purified immunoglobulin is covalently attached to a chromatographic resin such as CnBr-activated Sepharose (Pharmacia LKB Biotechnology). The antibody is coupled to the resin, the resin is blocked, and the derivative resin is washed according to the manufacturer's instructions.

Such immunoaffinity columns are utilized in the purification of CTGF by preparing a fraction from cells containing CTGF in a soluble form. This preparation is derived by solubilization of whole cells or of a subcellular fraction obtained via differential centrifugation (with or without addition of detergent) or by other methods well known in the art. Alternatively, soluble CTGF containing a signal sequence is secreted in useful quantity into the medium in which the cells are grown.

A soluble CTGF-containing preparation is passed over the immunoaffinity column, and the column is washed under conditions that allow the preferential absorbance of CTGF (e.g., high ionic strength buffers in the presence of detergent). Then, the column is eluted under conditions that disrupt antibody/protein binding (e.g., a buffer of pH 2-3 or a high concentration of a chaotrope such as urea or thiocyanate ion), and CTGF is collected.

### Example 8: Drug Screening

This invention is particularly useful for screening therapeutic compounds by using CTGF or fragments thereof in any of a variety of drug screening techniques.

The following example provides a system for drug screening measuring CTGF.

The recombinant protein-His fusion protein can be purified from the crude lysate by metal-affinity chromatography using Ni-NTA agarose. This allows the specific retention of the recombinant material (since this is fused to the His-tag) whilst the endogenous insect proteins are washed off. The recombinant material is then eluted by competition with imidazol.

CTGF protein expression in tissues, tissue homogenates and body fluids including plasma and serum can be measured by antibody-based strategies, e. g. by ELISA technology or Western Blotting /Immunfluorescence. A polyclonal antibody generated against the full-length CTGF has been described in the literature [Vehvilainen et al. (2003)].

### Example 9: Rational Drug Design

The goal of rational drug design is to produce structural analogs of biologically active polypeptides of interest or of small molecules with which they interact, agonists, antagonists, or inhibitors. Any of these examples are used to fashion drugs which are more active or stable forms of the polypeptide or which enhance or interfere with the function of a polypeptide in vivo.

In one approach, the three-dimensional structure of a protein of interest, or of a protein-inhibitor complex, is determined by x-ray crystallography, by computer modeling or, most typically, by a combination of the two approaches. Both the shape and charges of the polypeptide must be ascertained to elucidate the structure and to determine active site(s) of the molecule. Less often, useful information regarding the structure of a polypeptide is gained by modeling based on the structure of homologous proteins. In both cases, relevant structural information is used to design efficient inhibitors. Useful examples of rational drug design include molecules which have improved activity or stability or which act as inhibitors, agonists, or antagonists of native peptides.

It is also possible to isolate a target-specific antibody, selected by functional assay, as described above, and then to solve its crystal structure. This approach, in principle, yields a pharmacore upon which subsequent drug design is based. It is possible to bypass protein crystallography altogether by generating anti-idiotypic antibodies (anti-ids) to a functional, pharmacologically active antibody. As a mirror image of a mirror image, the binding site of the anti-ids is expected to be an analog of the original receptor. The anti-id is then used to identify and isolate peptides from banks of chemically or biologically produced peptides. The isolated peptides then act as the pharmacore.

By virtue of the present invention, sufficient amount of polypeptide are made available to perform such analytical studies as X-ray crystallography. In addition, knowledge of the CTGF amino acid sequence provided herein provides guidance to those employing computer modeling techniques in place of or in addition to x-ray crystallography.

### Example 10: Identification of Other Members of the Signal Transduction Complex

Labeled CTGF is useful as a reagent for the purification of molecules with which it interacts. In one embodiment of affinity purification, CTGF is covalently coupled to a chromatography column. Cell-free extract derived from synovial cells or putative target cells is passed over the column, and molecules with appropriate affinity bind to CTGF. CTGF-complex is recovered from the column, and the CTGF-binding ligand disassociated and subjected to N-terminal protein sequencing. The amino acid sequence information is then used to identify the captured molecule or to design degenerate oligonucleotide probes for cloning the relevant gene from an appropriate cDNA library. In an alternate method, antibodies are raised against CTGF, specifically monoclonal antibodies. The monoclonal antibodies are screened to identify those which inhibit the binding of labeled CTGF. These monoclonal antibodies are then used therapeutically.

### Example 11: Use and Administration of Antibodies, Inhibitors, or Antagonists

Antibodies, inhibitors, or antagonists of CTGF or other treatments and compunds that are limiters of signal transduction (LSTs), provide different effects when administered therapeutically. LSTs are formulated in a nontoxic, inert, pharmaceutically acceptable aqueous carrier medium preferably at a pH of about 5 to 8, more preferably 6 to 8, although pH may vary according to the characteristics of the antibody, inhibitor, or antagonist being formulated and the condition to be treated. Characteristics of LSTs include solubility of the molecule, its half-life and antigenicity/- immunogenicity. These and other characteristics aid in defining an effective carrier. Native human proteins are preferred as LSTs, but organic or synthetic molecules resulting from drug screens are equally effective in particular situations.

LSTs are delivered by known routes of administration including but not limited to topical creams and gels; transmucosal spray and aerosol; transdermal patch and bandage; injectable, intravenous and lavage formulations; and orally administered liquids and pills particularly formulated to resist stomach acid and enzymes. The particular formulation, exact dosage, and route of administration is determined by the attending physician and varies according to each specific situation.

Such determinations are made by considering multiple variables such as the condition to be treated, the LST to be administered, and the pharmacokinetic profile of a particular LST. Additional factors which are taken into account include severity of the disease state, patient's age, weight, gender and diet, time and frequency of LST administration, possible combination with other drugs, reaction sensitivities, and tolerance/response to therapy. Long acting LST formulations might be administered every 3 to 4 days, every week, or once every two weeks depending on half-life and clearance rate of the particular LST.

Normal dosage amounts vary from 0.1 to 10³ µg, up to a total dose of about 1 g, depending upon the route of administration. Guidance as to particular dosages and methods of delivery is provided in the literature; see U.S. Pat. Nos. 4,657,760; 5,206,344; or 5,225,212. Those skilled in the art employ different formulations for different LSTs. Administration to cells such as nerve cells necessitates delivery in a manner different from that to other cells such as vascular endothelial cells.

It is contemplated that abnormal signal transduction, trauma, or diseases which trigger CTGF activity are treatable with LSTs. These conditions or diseases are specifically diagnosed by the tests discussed above, and such testing should be performed in suspected cases of viral, bacterial or fungal infections, allergic responses, mechanical injury associated with trauma, hereditary diseases, lymphoma or carcinoma, or other conditions which activate the genes of lymphoid or neuronal tissues.

### Example 12: Production of Non-human Transgenic Animals

Animal model systems which elucidate the physiological and behavioral roles of the CTGF are produced by creating nonhuman transgenic animals in which the activity of the CTGF is either increased or decreased, or the amino acid sequence of the expressed CTGF is altered, by a variety of techniques. Examples of these techniques include, but are not limited to: 1) Insertion of normal or mutant versions of DNA encoding a CTGF, by microinjection, electroporation, retroviral transfection or other means well known to those skilled in the art, into appropriately fertilized embryos in order to produce a transgenic animal or 2) homologous recombination of mutant or normal, human or animal versions of these genes with the native gene locus in transgenic animals to alter the regulation of expression or the structure of these CTGF sequences. The technique of homologous recombination is well known in the art. It replaces the native gene with the inserted gene and hence is useful for producing an animal that cannot express native CTGFs but does express, for example, an inserted mutant CTGF, which has replaced the native CTGF in the animal's genome by recombination, resulting in underexpression of the transporter. Microinjection adds genes to the genome, but does not remove them, and the technique is useful for producing an animal which expresses its own and added CTGF, resulting in overexpression of the CTGF.

One means available for producing a transgenic animal, with a mouse as an example, is as follows: Female mice are mated, and the resulting fertilized eggs are dissected out of their oviducts. The eggs are stored in an appropriate medium such as cesiumchloride M2 medium. DNA or cDNA encoding CTGF is purified from a vector by methods well known to the one skilled in the art. Inducible promoters may be fused with the coding region of the DNA to provide an experimental means to regulate expression of the transgene. Alternatively or in addition, tissue specific regulatory elements may be fused with the coding region to permit tissue-specific expression of the transgene. The DNA, in an appropriately buffered solution, is put into a microinjection needle (which may be made from capillary tubing using a piper puller) and the egg to be injected is put in a depression slide. The needle is inserted into the pronucleus of the egg, and the DNA solution is injected. The injected egg is then transferred into the oviduct of a pseudopregnant mouse which is a mouse stimulated by the appropriate hormones in order to maintain false pregnancy, where it proceeds to the uterus, implants, and develops to term. As noted above, microinjection is not the only method for inserting DNA into the egg but is used here only for exemplary purposes.

### Example 13: Use of CTGF as a Biomarker, therapeutic and diagnostic Target in cardiovascular disease (DOCA)

The DOCA-salt hypertensive rat model is a well established model of left ventricular hypertrophy.

Uninephrectomized male Sprague-Dawley rats weighing 300-350g were given 1 % NaCl in drinking water and subcutaneous injections of deoxycorticosterone acetate (DOCA, 30mg/kg once weekly) for four weeks. Untreated rats without uninephrectomy served as control rats.

After four weeks DOCA-salt rats showed a significant increase in the tibia length-corrected left ventricular mass (DOCA-salt: 25,87 ± 0,84 mg/mm vs. control: 21,03 ± 0,60 mg/mm). At this time point heart and Li-Heparin plasma samples were taken for expression analysis.

Total cellular RNA was isolated with the Trizol-Reagent protocol according to the manufacturer's specifications (Invitrogen; USA). Total RNA prepared by the Trizol-reagent protocol was treated with DNAse I to remove genomic DNA contamination.

For relative quantitation of the mRNA distribution of CTGF, total RNA from each sample was first reverse transcribed. I µg of total RNA was reverse transcribed using ImProm-II Reverse Trascription System (Promega, USA) according to the manufactures protocol. The final volume was adjusted to 200 µl with water.

For relative quantitation of the distribution of CTGF mRNA the Applied Bioscience ABI 7900HT Sequence Detection system was used according to the manufacturer's specifications and protocols. PCR reactions were set up to quantitate CTGF and the housekeeping gene L32. Forward and reverse primers and probes for CTGF were designed using the Applied Bioscience ABI Primer Express^{™} software and were synthesized by Eurogentec (Belgium). The CTGF forward primer sequence was: Primer1 (SEQ ID NO: 5). The CTGF reverse primer sequence was Primer2 (SEQ ID NO: 7). Probe1 (SEQ ID NO: 6), labelled with FAM (carboxyfluorescein succinimidyl ester) as the reporter dye and TAMRA (carboxytetramethylrhodamine) as the quencher, is used as a probe for CTGF. The following reagents were prepared in a total of 20 µl : 1 x qPCR-MasterMix (Eurogentec; Belgium) and Probe1 (SEQ ID NO: 6), CTGF forward and reverse primers each at 200 nM, 200 nM CTGF FAM/TAMRA-labelled probe, and 5 µl of template cDNA. Thermal cycling parameters were 2 min at 50°C, followed by 10 min at 95°C, followed by 40 cycles of melting at 95°C for 15 sec and annealing/extending at 60°C for I min.

### Calculation of relative expression

The CT (threshold cycle) value is calculated as described in the "Quantitative determination of nucleic acids" section.
deltaCT =CTCTGF - CT132
relative expression = 2^(15-deltaCT)

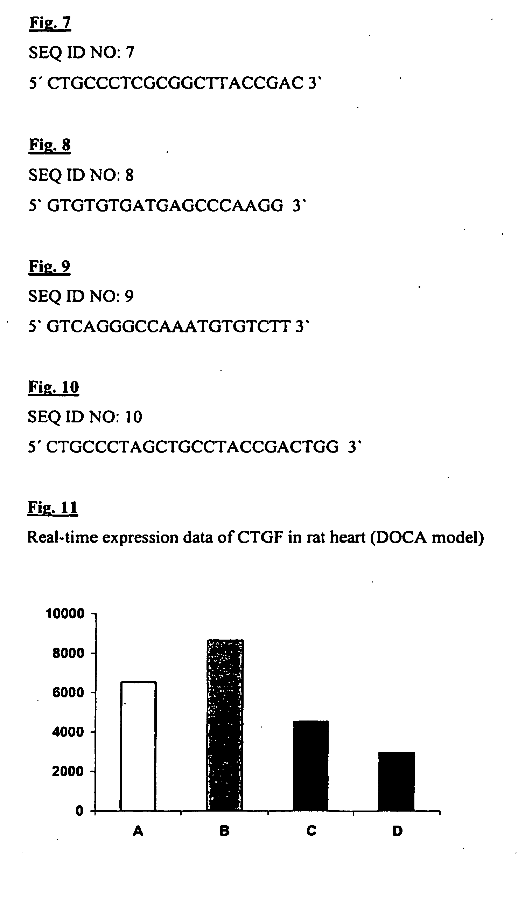

The results of the the mRNA-quantification (expression profiling) is shown in Figure 11.

### Example 14: Use of CTGF as a Biomarker, therapeutic and diagnostic Target in cardiovascular disease (Occlusion)

In the chronic myocardial infarction model in rat [Pfeffer et al, (1979)] left coronary artery ligation is performed under isoflurane anaesthesia. Following a left thoractomy at the fourth intercostal space, the pericardium is opened and the heart briefly exteriorized. The left coronary artery (LAD) is chronically ligated. In sham operated animals the LAD stays open. The chest is closed and animals are weaned from the ventilator and placed in cages with free access to food and water. One week after LAD occlusion application of test compounds is started. Heart tissue and plasma samples are analyzed 9 weeks after induction of the infarct towards plasma markers and expression profiles.

Total cellular RNA was isolated with the Trizol-Reagent protocol according to the manufacturer's specifications (Invitrogen; USA). Total RNA prepared by the Trizol-reagent protocol was treated with DNAse I to remove genomic DNA contamination.

For relative quantitation of the mRNA distribution of CTGF, total RNA from each sample was first reverse transcribed. 1 µg of total RNA was reverse transcribed using ImProm-II Reverse Trascription System (Promega, USA) according to the manufactures protocol. The final volume was adjusted to 200 µl with water.

For relative quantitation of the distribution of CTGF mRNA the Applied Bioscience ABI 7900HT Sequence Detection system was used according to the manufacturer's specifications and protocols. PCR reactions were set up to quantitate CTGF and the housekeeping gene L32. Forward and reverse primers and probes for CTGF were designed using the Applied Bioscience ABI Primer Express^{™} software and were synthesized by Eurogentec (Belgium). The CTGF forward primer sequence was: Primer1 (SEQ ID NO: 5). The CTGF reverse primer sequence was Primer2 (SEQ ID NO: 7). Probe1 (SEQ ID NO: 6), labelled with FAM (carboxyfluorescein succinimidyl ester) as the reporter dye and TAMRA (carboxytetramethylrhodamine) as the quencher, is used as a probe for CTGF. The following reagents were prepared in a total of 20 µl : 1 x qPCR-MasterMix (Eurogentec; Belgium) and Probe1 (SEQ ID NO: 6), CTGF forward and reverse primers each at 200 nM, 200 nM CTGF FAM/TAMRA-labelled probe, and 5 µl of template cDNA. Thermal cycling parameters were 2 min at 50°C, followed by 10 min at 95°C, followed by 40 cycles of melting at 95°C for 15 sec and annealing/extending at 60°C for I min.

### Calculation of relative expression

The CT (threshold cycle) value is calculated as described in the "Quantitative determination of nucleic acids" section.
deltaCT =CTCTGF - CT132
relative expression = 2^(15-deltaCT)

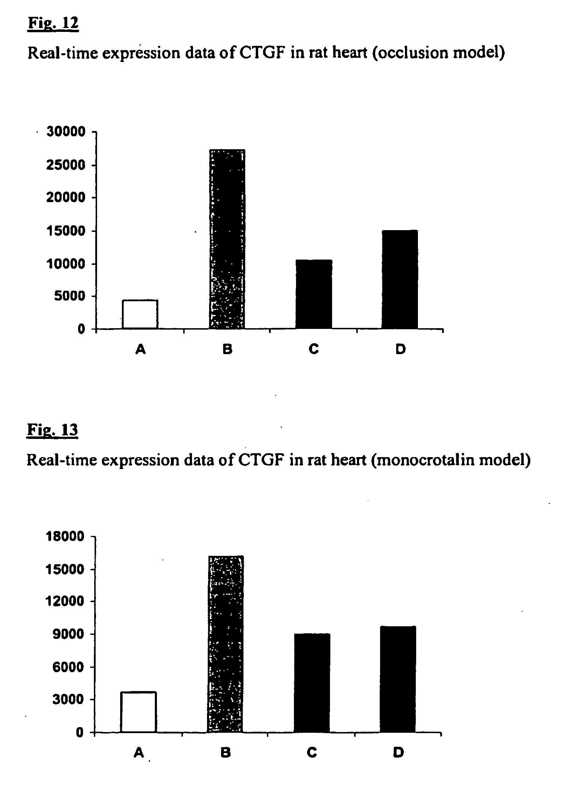

The results of the the mRNA-quantification (expression profiling) is shown in Figure 12.

### Example 15: Use of CTGF as a Biomarker, therapeutic and diagnostic Target in cardiovascular disease (Monocrotalin)

Adult male Sprague-Dawley rats weighing 250 to 300 g were given a single subcutaneous injection of either 60 mg/kg Monocrotaline or vehicle.

The Monocrotaline (MCT)-treated rat is a widely used animal model for pulmonary arterial hypertension. After subcutaneous injection the pyrrolizidine alkaloid MCT is activated by the liver to the toxic MCT pyrrole, which causes endothelial injury in the pulmonary vasculature within few days with subsequent remodeling of small pulmonary arteries (de novo muscularization and medial hypertrophy). In the present study, MCT induced severe, progressive pulmonary hypertension in all animals.

Four weeks after a single MCT injection, the rats displayed threefold elevated right ventricular systolic pressure (placebo MCT: 77,62 ± 4,17 mmHg vs. control: 26,4 ± 1,12 mmHg; mean ± sem), accompanied by a reduction of systemic arterial pressure, cardiac index, arterial oxygenation and central venous oxygen saturation. In accordance with these results, an impressive right heart hypertrophy was observed (right ventricle/left ventricle + septum ratio placebo MCT: 0,62 ± 0,03 vs. control: 0,26 ± 0,01).

Heart and Li-Heparin plasma samples were taken for expression analysis four weeks after the MCT injection.

Total cellular RNA was isolated with the Trizol-Reagent protocol according to the manufacturer's specifications (Invitrogen; USA). Total RNA prepared by the Trizol-reagent protocol was treated with DNAse I to remove genomic DNA contamination.

For relative quantitation of the mRNA distribution of CTGF, total RNA from each sample was first reverse transcribed. 1 µg of total RNA was reverse transcribed using ImProm-II Reverse Trascription System (Promega, USA) according to the manufactures protocol. The final volume was adjusted to 200 µl with water.

For relative quantitation of the distribution of CTGF mRNA the Applied Bioscience ABI 7900HT Sequence Detection system was used according to the manufacturer's specifications and protocols. PCR reactions were set up to quantitate CTGF and the housekeeping gene L32. Forward and reverse primers and probes for CTGF were designed using the Applied Bioscience ABI Primer Express^{™} software and were synthesized by Eurogentec (Belgium). The CTGF forward primer sequence was: Primer1 (SEQ ID NO: 5). The CTGF reverse primer sequence was Primer2 (SEQ ID NO: 7). Probe1 (SEQ ID NO: 6), labelled with FAM (carboxyfluorescein succinimidyl ester) as the reporter dye and TAMRA (carboxytetramethylrhodamine) as the quencher, is used as a probe for CTGF. The following reagents were prepared in a total of 20 µl : 1 x qPCR-MasterMix (Eurogentec; Belgium) and Probe1 (SEQ ID NO: 6), CTGF forward and reverse primers each at 200 nM, 200 nM CTGF FAM/TAMRA-labelled probe, and 5 µl of template cDNA. Thermal cycling parameters were 2 min at 50°C, followed by 10 min at 95°C, followed by 40 cycles of melting at 95°C for 15 sec and annealing/extending at 60°C for 1 min.

### Calculation of relative expression

The CT (threshold cycle) value is calculated as described in the "Quantitative determination of nucleic acids" section.
deltaCT =CTCTGF - CT132
relative expression = 2^(15-deltaCT)

The results of the the mRNA-quantification (expression profiling) is shown in Figure 13.

### Example 16: Microarray experiments

Total RNA extracted from cardiac tissue and was purified using an affinity resin column (RNeasy; Qiagen, Hilden, Germany), quantified by spectrophotometry (absorbance 260 nm), and the quality of RNA was assessed by microfluidics electrophoretical separation with a Bioanalyzer (Agilent Technologies, Palo Alto, USA). Purified total RNA (1 µg) was converted to cDNA using the Superscript Choice cDNA synthesis kit (Invitrogen, Carlsbad, CA, USA), incorporating a T7-(dT)24 primer. Double-stranded cDNA was then purified by affinity resin column (Clean up Kit, Qiagen, Hilden, Germany) with ethanol extraction. Purified cDNA was used as a template for in vitro transcription reaction for the synthesis of biotinylated cRNA using an Enzo BioArray HighYield RNA transcription labeling kit (Affymetrix, Santa Clara, CA), and further purified using an affinity resin column (Clean up Kit, Qiagen, Hilden, Germany). After purification, in vitro cRNA was fragmented in buffer containing magnesium at 95°C for 35 min. Fragmented cRNA was hybridized onto the Affymetrix GeneChip Human Genome U133 Plus 2.0 Array. Briefly, 15 µg fragmented cRNA was added along with control cRNA (BioB, BioC, and BioD), herring sperm DNA (10 mg/ml), 10% DMSO, and acetylated BSA (50 mg/ml) to the hybridization buffer. The hybridization mixture was heated at 99°C for 5 min, incubated at 45°C for 5 min, centrifuged for 5 min at 13.000 rpm, and injected into the microarray. After hybridization at 45°C for 16 h rotating at 60 rpm, the array was washed and stained with the Affymetrix Fluidics Protocols-antibody amplification for Eukaryotic Targets, and scanned using an Affymetrix microarray scanner (GeneChip Scanner 3000 7G system) at 570 nm.

### Example 17: Microarray expression data from human heart of CHF patients with left ventricular assist devices

Implantation of left ventricular assist devices (LVAD) often is the only possible means of supporting patients with end-stage heart failure in the form of bridging to transplantation (see [Clegg et al. (2005)] for a review). Like the heart, the LVAD is a pump. One end hooks up to the left ventricle - that's the chamber of the heart that pumps blood out of the lungs and into the body. The other end hooks up to the aorta, the body's main artery. A tube passes from the device through the skin. The outside of the tube is covered with a special material to aid in healing and allow the skin to regrow. The LVAD is implanted during open-heart surgery. Recent reports demonstrate that LVAD support may be associated with adaptive remodeling of the ventricular myocardium, including reduced LV mass, wall thickness and myocyte diameter, changes in LV pressure-volume relationships and reversal of LV chamber dilation [Li et al. (2001)].

Myocardial samples of the left ventricle were collected during cardiac surgery from 32 heart failure patients at the time of cardiac transplantation or insertion of a mechanical assist device. Corresponding myocardial specimen are designed as pre- and post-LVAD samples. All procedures involving human tissue use were approved by the institutional review boards of the "Heart- and Diabetes-Center North Rhine Westphalia, Bad Oeynhausen, Germany. Consent was obtained from patients before tissue harvest. Samples were immediately frozen in liquid nitrogen and pulverized using pestle and mortar. Total RNA was isolated according to standard procedures.

### Example 18: Data analysis from microarray experiments

Raw data analysis and scaling were performed in Microarray Suite 5.0 software (Affymetrix), and normalization and further analysis in expressionist Pro 3.0 (Genedata). Results for HG-U133 Plus 2.0 arrays were subjected to global scaling with a target intensity of 100.

Base-2 logarithms were calculated for all expression values and taken for subsequent statistical analysis. To analyze the differential expression between the two groups, non-failing hearts (N) and pre-operation hearts (P), a two-tailed Student's test was applied to the expression values under the assumption of equal variances. A resultant p-value of less or equal than 0,05 was taken as indicator for significant differential expression.

### References

EP 1 069 188
EP 1 275 733
EP 1 308 459
EP 1 560 025
EP 1 612 281
U.S. 4,522,811
U.S. 5,057,414
U.S. 5,283,317
U.S. 5,565,332
U.S. 5,723,323.
U.S. 5,747,334
U.S. 5,783,384
U.S. 5,885,814
U.S. 5,985,629
WO 84/03564
WO 3/03151
WO 94/13804
WO 00/47750
WO 02/06492
WO 02/26958
WO 02/47670
WO 03/051370
WO 02/081745
Altschul SF, Madden TL, Schaffer AA, Zhang J, Zhang Z, Miller W, Lipman DJ; Nucleic Acids Res 1997 Sep 1; 25(17): 3389-402
Appa Rao et al., 1997, Protein Expr Purif Nov, 11(2): 201-8
Alpert, J. S., et al. J. Am. Coll. Cardiol. 2000; 36:959-69
Avalle et al., Ann. N Y Acad.Sci. 864:118 (1998)).
Barnes, 2000, Chest, 117:10S14S
Barrett et al., (Eds.), Handbook of Proteolytic Enzymes (Academic Press Inc. 1998).
Barrett (Ed.), Methods in Enzymology, Proteolytic Enzymes: Serine and Cysteine Peptidases
(Academic Press Inc. 1994)
Boersma, E., et al. Lancet 2002; 359:189-98
Bork P. , 1993, FEBS Lett;327:125-30.
Botstein et al., 1980, Am J Hum Genet. 32: 314-31
Clegg et al. 2005, Health Technol Assess. Nov; 9(45):1-148
Colbere-Garapin et al., 1981, J. Mol. Biol. 150, 1-14
Chen CC, Chen N, Lau LF., 2001, J Biol Chem;276:10443-52.
Christenson, R. H., et al., Clin. Chem. 2001; 47:464-470
Cunningham and Wells, J. Mol. Biol. 234:554 (1993).
DesGroseillers et al. (2001), DNA Cell Biol. Aug;20(8):493-8.
de Lemos, J. A., et al. J. Am. Coll. Cardiol. 2002; 40:238-44
Engelhard et al., 1994, Proc. Nat. Acad. Sci. 91, 3224-3227
Friboulet et al., Appl. Biochem. Biotechnol. 47:229 (1994)
Gao R, Brigstock DR., Hepatol Res 2003;27:214-20.
Gao R, Brigstock DR., J Biol Chem 2004;279:8848-55.
Gergen and Weiss , 1992, Am Rev Respir Dis 146:823-824
Gibson et al., 1996, Genome Research 6: 995-1001
Grotendorst GR, Okochi H, Hayashi N., Cell Growth Differ 1996;7:469-80.
Haseloff et al., 1988 , Nature 334, 585-591
Heid et al., 1996, Genome Research 6: 986-994
Holland et al., 1991, PNAS 88: 7276-7280
Ifon et al. 2005, Cancer Cell Int. Jun 22;5:19.
Igarashi A, Okochi H, Bradham DM, Grotendorst GR., 1993, Mol Biol Cell;4:637-45. Jedsadayanmata A, Chen CC, Kireeva ML, Lau LF, Lam SC., J Biol Chem 1999;274:24321-7.
Jeffreys et al., 1985, Nature 316: 76-9
Johnson et al., 1989, Endoc. Rev. 10, 317-331
Joron et al., Ann. N Y Acad. Sci. 672:216 (1992)
Karlsson, Immunol. Methods 145:229 (1991)
Kellogg et al., 1990, Anal. Biochem. 189:202-208
Lam , 1997, Anticancer Drug Res. 12(3):145-67
Li et al. 2001, Circulation. Sep 4;104(10):1147-52
Livak et al., 1995 , PCR Methods and Applications 357-362
Logan, Shenk, 1984, Proc. Natl. Acad. Sci. 81, 3655-3659
Lowy et al., 1980, Cell 22, 817-23
Maddox et al., 1983, J. Exp. Med. 158, 1211-1216
McConnell et al., 1992 , Science 257, 1906-1912
Mercurio S, Latinkic B, Itasaki N, Krumlauf R, Smith JC., Development 2004;131:2137-47.
Monfardini et al., Proc. Assoc. Am. Physicians 108:420 (1996)
Nicholls et al., 1993, J. Immunol. Meth. 165, 81-91
Newby, L. K., et al. Circulation 2001:103; 1832-7
Pentecost et al. 2005, Mol Cell Endocrinol. Jun 30;238(1-2):9-25.
Piatak et al., 1993, BioTechniques 14:70-81
Piatak et al., 1993, Science 259:1749-1754
Porath et al., 1992, Prot. Exp. Purif. 3, 263-281
Pfeffer et al., Circ Res. 1979 Apr;44(4):503-12.
Roberge et al., 1995, Science 269, 202-204
Roestenberg P, van Nieuwenhoven FA, Wieten L, et al., Diabetes Care 2004;5:1164-70.
Sagnella, G. A., Clinical Science 95:519-529, 1998
Schober JM, Chen N, Grzeszkiewicz TM, et al., Blood 2002;99:4457-65.
Scott and Smith (1990) Science 249:386-390
Segarini PR, Nesbitt JE, Li D, Hays LG, Yates JR III, Carmichael DF., J Biol Chem 2001; 276:40659-67.
Shimo T, Kubota S, Kondo S, et al., Cancer Lett 2001; 174:57-64.
Sjolander, Urbaniczky, 1991, Anal. Chem. 63, 2338-2345
Suzuma K, Naruse K, Suzuma I, et al., Chem 2000;275:40725-31.
Szabo et al., 1995, Curr. Opin. Struct. Biol. 5, 699-705
Takigawa M., Drug News Perspect 2003;16:11-21.
Thomas, 1980, Proc. Nat. Acad. Sci., 77:5201-5205
Uhlmann et al., 1987, Tetrahedron. Lett. 215, 3539-3542
Weber et al., 1990, Genomics 7: 524-30
Wigler et al., 1977, Cell 11, 223-32
Wigler et al., 1980, Proc. Natl. Acad Sci. 77, 3567-70

## Claims

1. A method of use of CTGF as a disease, efficacy or surrogate endpoint biomarker for a therapy of pulmonary hypertension comprising :
i) obtaining a baseline level of CTGF in biological sample from a diseased mammal,
ii) measuring the level of CTGF in the one or more subsequent biological samples taken from the diseased mammal receiving treatment for the disease,
iii) comparing the level of CTGF in the one or more subsequent biological samples with the baseline sample, and
iv) determining whether increased dosages, additional or alternative treatments are necessary based on CTGF levels obtained from one or more subsequent biological samples compared to the baseline CTGF level,
wherein the sample is a heart tissue sample.

2. A method of claim 1 wherein the level of CTGF is determined by determining the level of CTGF polynucleotide.

3. A method of claim 1 wherein the level of CTGF is determined by determining the level of CTGF polypeptide.

4. A method of claim 1 wherein the level of CTGF is determined by determining the level of CTGF activity.

5. A method of claim 1 wherein the mammal is a human.

6. A method of claim 1 wherein the use of CTGF is combined with the use of one or more biomarkers.

7. A method of claim 1 wherein the use of CTGF is combined with the use of one or more biomarkers which are comprised in a group of biomarkers consisting of CRTAC, PRSS23, FN1, LTBP2, TGFB2, and NPR3.

8. A method of claim 1 wherein the use of CTGF is combined with the use of one or more biomarkers which are comprised in a group of biomarkers consisting of BNP, ANP, Troponin, CRP, Myoglobin, CK-MB or metabolites.

9. A method of claim 1 wherein the use of CTGF is combined with the use of one or more clinical biomarkers which are comprised in a group of biomarkers consisting of blood pressure, heart rate, pulmonary artery pressure, or systemic vascular resistance.

## Patentansprüche

1. Verfahren zur Verwendung von CTGF als Erkrankungs-, Wirksamkeits- oder Surrogat-Endpunkt-Biomarker für eine Therapie von Lungenhochdruck, umfassend:
i) Gewinnen eines Grundlinienspiegels von CTGF in einer biologischen Probe aus einem erkrankten Säuger,
ii) Messen des Spiegels von CTGF in einer oder mehreren nachfolgenden biologischen Proben, die von dem erkrankten Säuger, der Behandlung für die Erkrankung erhält, genommen werden,
iii) Vergleichen des Spiegels von CTGF in der einen oder den mehreren nachfolgenden biologischen Proben mit der Grundlinienprobe, und
iv) Bestimmen, ob auf der Grundlage der CTGF-Spiegel, die aus der einen oder den mehreren nachfolgenden biologischen Proben erhalten wurden, im Vergleich zu dem Grundlinien-CTGF-Spiegel erhöhte Dosierungen, zusätzliche oder alternative Behandlungen erforderlich sind,
wobei die Probe eine Herzgewebeprobe ist.

2. Verfahren gemäß Anspruch 1, wobei der Spiegel von CTGF durch Bestimmen des Spiegels von CTGF-Polynucleotid bestimmt wird.

3. Verfahren gemäß Anspruch 1, wobei der Spiegel von CTGF durch Bestimmen des Spiegels von CTGF-Polypeptid bestimmt wird.

4. Verfahren gemäß Anspruch 1, wobei der Spiegel von CTGF durch Bestimmen der Höhe von CTGF-Aktivität bestimmt wird.

5. Verfahren gemäß Anspruch 1, wobei der Säuger ein Mensch ist.

6. Verfahren gemäß Anspruch 1, wobei die Verwendung von CTGF mit der Verwendung von einem oder mehreren Biomarkern kombiniert wird.

7. Verfahren gemäß Anspruch 1, wobei die Verwendung von CTGF mit der Verwendung von einem oder mehreren Biomarkern kombiniert wird, die in der Gruppe von Biomarkern bestehend aus CRTAC, PRSS23, FN1, LTBP2, TGFB2 und NPR3 enthalten sind.

8. Verfahren gemäß Anspruch 1, wobei die Verwendung von CTGF mit der Verwendung von einem oder mehreren Biomarkern kombiniert wird, die in der Gruppe von Biomarkern bestehend aus BNP, ANP, Troponin, CRP, Myoglobin, CK-MB oder Metaboliten enthalten sind.

9. Verfahren gemäß Anspruch 1, wobei die Verwendung von CTGF mit der Verwendung von einem oder mehreren klinischen Biomarkern kombiniert wird, die in der Gruppe von Biomarkern bestehend aus Blutdruck, Herzfrequenz, Lungenarteriendruck und systemischem Gefäßwiderstand enthalten sind.

## Revendications

1. Procédé d'utilisation de CTGF en tant que biomarqueur de critère de maladie, d'efficacité ou de succédané pour une thérapie d'hypertension pulmonaire comprenant :
i) l'obtention d'un niveau de référence de CTGF dans un échantillon biologique d'un mammifère malade,
ii) la mesure du niveau de CTGF dans les un ou plusieurs échantillons biologiques successifs prélevés sur le mammifère malade recevant un traitement pour la maladie,
iii) la comparaison du taux de CTGF dans les un ou plusieurs échantillons biologiques successifs à l'échantillon de référence, et
iv) la détermination si des doses augmentées, des traitements additionnels ou autres sont nécessaires sur la base des taux de CTGF obtenus à partir d'un ou plusieurs échantillons biologique successifs comparés au taux de CTGF de référence,
l'échantillon étant un échantillon de tissu cardiaque.

2. Procédé de la revendication 1 dans lequel le taux de CTGF est déterminé en déterminant le taux de polynucléotide CTGF.

3. Procédé de la revendication 1 dans lequel le taux de CTGF est déterminé en déterminant le taux de polypeptide CTGF.

4. Procédé de la revendication 1 dans lequel le taux de CTGF est déterminé en déterminant le taux d'activité CTGF.

5. Procédé de la revendication 1 dans lequel le mammifère est un humain.

6. Procédé de la revendication 1 dans lequel l'utilisation de CTGF est combinée avec l'utilisation d'un ou plusieurs biomarqueurs.

7. Procédé de la revendication 1 dans lequel l'utilisation de CTGF est combinée avec l'utilisation d'un ou plusieurs biomarqueurs qui sont compris dans un groupe de biomarqueurs constitué de CRTAC, PRSS23, FN1, LTBP2, TGFB2, et NPR3.

8. Procédé de la revendication 1 dans lequel l'utilisation de CTGF est combinée avec l'utilisation d'un ou plusieurs biomarqueurs qui sont compris dans un groupe de biomarqueurs constitué de BNP, ANP, la troponine, CRP, la myoglobine, CK-MB ou des métabolites.

9. Procédé de la revendication 1 dans lequel l'utilisation de CTGF est combinée avec l'utilisation d'un ou plusieurs biomarqueurs cliniques qui sont compris dans un groupe de biomarqueurs constitué de la pression artérielle, la fréquence cardiaque, la pression artérielle pulmonaire, ou la résistance vasculaire systémique.


### SEQUENCE LISTING

<110> Bayer HealthCare AG
<120> CTGF as a Biomarker, therapeutic and diagnostic Target
   <130> BHC 06 1 150
   <160> 10
   <170> PatentIn version 3.3
   <210> 1
   <211> 2312
   <212> DNA
   <213> Homo sapiens
   <400> 1
   tccagtgacg gagccgcccg gccgacagcc ccgagacgac agcccggcgc gtcccggtcc     60
   ccacctccga ccaccgccag cgctccaggc cccgcgctcc ccgctcgccg ccaccgcgcc    120
   ctccgctccg cccgcagtgc caaccatgac cgccgccagt atgggccccg tccgcgtcgc    180
   cttcgtggtc ctcctcgccc tctgcagccg gccggccgtc ggccagaact gcagcgggcc    240
   gtgccggtgc ccggacgagc cggcgccgcg ctgcccggcg ggcgtgagcc tcgtgctgga    300
   cggctgcggc tgctgccgcg tctgcgccaa gcagctgggc gagctgtgca ccgagcgcga    360
   cccctgcgac ccgcacaagg gcctcttctg tgacttcggc tccccggcca accgcaagat    420
   cggcgtgtgc accgccaaag atggtgctcc ctgcatcttc ggtggtacgg tgtaccgcag    480
   cggagagtcc ttccagagca gctgcaagta ccagtgcacg tgcctggacg gggcggtggg    540
   ctgcatgccc ctgtgcagca tggacgttcg tctgcccagc cctgactgcc ccttcccgag    600
   gagggtcaag ctgcccggga aatgctgcga ggagtgggtg tgtgacgagc ccaaggacca    660
   aaccgtggtt gggcctgccc tcgcggctta ccgactggaa gacacgtttg gcccagaccc    720
   aactatgatt agagccaact gcctggtcca gaccacagag tggagcgcct gttccaagac    780
   ctgtgggatg ggcatctcca cccgggttac caatgacaac gcctcctgca ggctagagaa    840
   gcagagccgc ctgtgcatgg tcaggccttg cgaagctgac ctggaagaga acattaagaa    900
   gggcaaaaag tgcatccgta ctcccaaaat ctccaagcct atcaagtttg agctttctgg    960
   ctgcaccagc atgaagacat accgagctaa attctgtgga gtatgtaccg acggccgatg   1020
   ctgcaccccc cacagaacca ccaccctgcc ggtggagttc aagtgccctg acggcgaggt   1080
   catgaagaag aacatgatgt tcatcaagac ctgtgcctgc cattacaact gtcccggaga   1140
   caatgacatc tttgaatcgc tgtactacag gaagatgtac ggagacatgg catgaagcca   1200
   gagagtgaga gacattaact cattagactg gaacttgaac tgattcacat ctcatttttc   1260
   cgtaaaaatg atttcagtag cacaagttat ttaaatctgt ttttctaact gggggaaaag    1320
   attcccaccc aattcaaaac attgtgccat gtcaaacaaa tagtctatct tccccagaca   1380
   ctggtttgaa gaatgttaag acttgacagt ggaactacat tagtacacag caccagaatg   1440
   tatattaagg tgtggcttta ggagcagtgg gagggtacca gcagaaaggt tagtatcatc   1500
   agatagctct tatacgagta atatgcctgc tatttgaagt gtaattgaga aggaaaattt   1560
   tagcgtgctc actgacctgc ctgtagcccc agtgacagct aggatgtgca ttctccagcc   1620
   atcaagagac tgagtcaagt tgttccttaa gtcagaacag cagactcagc tctgacattc   1680
   tgattcgaat gacactgttc aggaatcgga atcctgtcga ttagactgga cagcttgtgg   1740
   caagtgaatt tcctgtaaca agccagattt tttaaaattt atattgtaaa tattgtgtgt   1800
   gtgtgtgtgt gtgtatatat atatatatat gtacagttat ctaagttaat ttaaagttgt   1860
   ttgtgccttt ttatttttgt ttttaatgct ttgatatttc aatgttagcc tcaatttctg   1920
   aacaccatag gtagaatgta aagcttgtct gatcgttcaa agcatgaaat ggatacttat   1980
   atggaaattc tctcagatag aatgacagtc cgtcaaaaca gattgtttgc aaaggggagg   2040
   catcagtgtc cttggcaggc tgatttctag gtaggaaatg tggtagctca cgctcacttt   2100
   taatgaacaa atggccttta ttaaaaactg agtgactcta tatagctgat cagttttttc    2160
   acctggaagc atttgtttct actttgatat gactgttttt cggacagttt atttgttgag   2220
   agtgtgacca aaagttacat gtttgcacct ttctagttga aaataaagta tattttttct    2280
   aaaaaaaaaa aaaaacgaca gcaacggaat tc                                 2312
<210> 2
   <211> 2349
   <212> DNA
   <213> Rattus norvegicus
   <400> 2
   cacagctctt ctctccaaga agactcagcc agacccactc cagctccgac cctaggagac      60
   cgacctcctc cagacggcag cagccccagc ccagtggaca accccaggag ccaccacctg     120
   gagcgtccgg acaccaacct ccgccccgag accgagtcca ggctccggcc gcgcccctcg     180
   tcgcctctgc accccgctgt gcgtcctcct gccgcgcccc gaccatgctc gcctccgtcg     240
   cgggtcccgt tagcctcgcc ttggtgctcc tcctctgcac ccggcctgcc accggccagg     300
   actgcagcgc gcagtgtcag tgcgcagctg aagcggcgcc gcgctgcccc gccggcgtga     360
   gcctggtgct ggacggctgc ggctgctgcc gcgtctgcgc caagcagctg ggagaactgt     420
   gcacggagcg tgatccctgc gacccacaca agggtctctt ctgcgacttc ggctcccccg     480
   ccaaccgcaa gattggcgtg tgcactgcca aagatggtgc accctgtgtc ttcggtgggt     540
   ccgtgtaccg cagcggcgag tccttccaaa gcagttgcaa ataccagtgc acttgcctgg     600
   atggggccgt gggctgtgtg cccctgtgca gcatggacgt gcgcctgccc agccctgact     660
   gccccttccc gagaagggtc aagctgcccg ggaaatgctg tgaggagtgg gtgtgtgatg      720
   agcccaagga ccgcacagtg gttggccctg ccctagctgc ctaccgactg gaagacacat     780
   ttggccctga cccaactatg atgcgagcca actgcctggt ccagaccaca gagtggagcg     840
   cctgttctaa gacctgtggg atgggcatct ccacccgggt taccaatgac aataccttct     900
   gcaggctgga gaagcagagt cgtctctgca tggtcaggcc ctgtgaagct gacctagagg     960
   aaaacattaa gaagggcaaa aagtgcatcc ggacgcctaa aattgccaag cctgtcaagt    1020
   ttgagctttc tggctgcacc agtgtgaaga cctaccgggc taagttctgt ggggtgtgca    1080
   cggacggccg ctgctgcaca ccgcacagaa ccaccacact gccggtggag ttcaagtgcc    1140
   ccgatggcga gatcatgaaa aagaacatga tgttcatcaa gacctgtgcc tgccattaca    1200
   actgtcccgg ggacaatgac atctttgagt ccttgtacta caggaagatg tatggagaca    1260
   tggcgtaaag ccagggagta agggacacga actcatttag actataactt gaactgagtt    1320
   acatctcatt ttcttctgta aaaaaacaaa aaggattaca gtagcacatt aatttaaatc    1380
   tgggttccta actgctgtgg gagaaaacac cccaccgaag tgagaaccgt gtgtcattgt    1440
   catgcaaata gcctgtcaat ctcagacact ggtttcgaga cagtttagac ttgacagttg    1500
   ttcactagcg cacagtgaca gaacgcacac taaggtgagc ctcctggaag agtggagatg    1560
   ccaggagaaa gacaggtact agctgaggtc attttaaaag cagcgatatg cctacttttt    1620
   ggagtgtgac aggggaggga cattatagct tgcttgcaga cagacctgct ctagcaagag    1680
   ctgggtgtgt gtcctccact cggtgaggct gaagccagct attctttcag taagaacagc    1740
   agtttcagcg ctgacattct gattccagtg acactggtcg ggagtcagaa ccttgtctat    1800
   tagactggac agcttgtggc aagtgaattt gccggtaaca agccagattt ttatggatct    1860
   tgtaaatatt gtggataaat atatatattt gtacagttat ctaagttaat ttaaagacgt    1920
   ttgtgcctat tgttcttgtt ttaagtgctt ttggaatttt taaactgata gcctcaaact    1980
   ccaaacacca tcgataggac ataaagcttg tctgtgattc aaaacaaagg agatactgca    2040
   gtggaaactg taacctgagt gactgtctgt cagaacatat ggtacgtaga cggtaaagca    2100
   atggatcaga agtcagattt ctagtaggaa atgtaaaatc actgttggcg aacaaatggc    2160
   ctttattaag aaatggcttg ctcagggtaa ctggtcagat ttccacgagg aagtgtttgc    2220
   tgcttctttg actatgactg gtttgggagg cagtttattt gttgagagtg tgaccaaaag    2280
   ttacatgttt gcacctttct agttgaaaat aaagtatata tattttttat atgaaaaaaa    2340
   aaaaaaaaa                                                            2349
<210> 3
   <211> 349
   <212> PRT
   <213> Homo sapiens
   <400> 3
   Met Thr Ala Ala Ser Met Gly Pro Val Arg Val Ala Phe Val Val Leu
   1 5 10 15
   Leu Ala Leu Cys Ser Arg Pro Ala Val Gly Gln Asn Cys Ser Gly Pro
   20 25 30
   Cys Arg Cys Pro Asp Glu Pro Ala Pro Arg Cys Pro Ala Gly Val Ser
   35 40 45
   Leu Val Leu Asp Gly Cys Gly Cys Cys Arg Val Cys Ala Lys Gln Leu
   50 55 60
   Gly Glu Leu Cys Thr Glu Arg Asp Pro Cys Asp Pro His Lys Gly Leu
   65 70 75 80
   Phe Cys Asp Phe Gly Ser Pro Ala Asn Arg Lys Ile Gly Val Cys Thr
   85 90 95
   Ala Lys Asp Gly Ala Pro Cys Ile Phe Gly Gly Thr Val Tyr Arg Ser
   100 105 110
   Gly Glu Ser Phe Gln Ser Ser Cys Lys Tyr Gln Cys Thr Cys Leu Asp
   115 120 125
   Gly Ala Val Gly Cys Met Pro Leu Cys Ser Met Asp Val Arg Leu Pro
   130 135 140
   Ser Pro Asp Cys Pro Phe Pro Arg Arg Val Lys Leu Pro Gly Lys Cys
   145 150 155 160
   Cys Glu Glu Trp Val Cys Asp Glu Pro Lys Asp Gln Thr Val Val Gly
   165 170 175
   Pro Ala Leu Ala Ala Tyr Arg Leu Glu Asp Thr Phe Gly Pro Asp Pro
   180 185 190
   Thr Met Ile Arg Ala Asn Cys Leu Val Gln Thr Thr Glu Trp Ser Ala
   195 200 205
   Cys Ser Lys Thr Cys Gly Met Gly Ile Ser Thr Arg Val Thr Asn Asp
   210 215 220
   Asn Ala Ser Cys Arg Leu Glu Lys Gln Ser Arg Leu Cys Met Val Arg
   225 230 235 240
   Pro Cys Glu Ala Asp Leu Glu Glu Asn Ile Lys Lys Gly Lys Lys Cys
   245 250 255
   Ile Arg Thr Pro Lys Ile Ser Lys Pro Ile Lys Phe Glu Leu Ser Gly
   260 265 270
   Cys Thr Ser Met Lys Thr Tyr Arg Ala Lys Phe Cys Gly Val Cys Thr
   275 280 285
   Asp Gly Arg Cys Cys Thr Pro His Arg Thr Thr Thr Leu Pro Val Glu
   290 295 300
   Phe Lys Cys Pro Asp Gly Glu Val Met Lys Lys Asn Met Met Phe Ile
   305 310 315 320
   Lys Thr Cys Ala Cys His Tyr Asn Cys Pro Gly Asp Asn Asp Ile Phe
   325 330 335
   Glu Ser Leu Tyr Tyr Arg Lys Met Tyr Gly Asp Met Ala
   340 345
<210> 4
   <211> 347
   <212> PRT
   <213> Rattus norvegicus
   <400> 4
   Met Leu Ala Ser Val Ala Gly Pro Val Ser Leu Ala Leu Val Leu Leu
   1 5 10 15
   Leu Cys Thr Arg Pro Ala Thr Gly Gln Asp Cys Ser Ala Gln Cys Gln
   20 25 30
   Cys Ala Ala Glu Ala Ala Pro Arg Cys Pro Ala Gly Val Ser Leu Val
   35 40 45
   Leu Asp Gly Cys Gly Cys Cys Arg Val Cys Ala Lys Gln Leu Gly Glu
   50 55 60
   Leu Cys Thr Glu Arg Asp Pro Cys Asp Pro His Lys Gly Leu Phe Cys
   65 70 75 80
   Asp Phe Gly Ser Pro Ala Asn Arg Lys Ile Gly Val Cys Thr Ala Lys
   85 90 95
   Asp Gly Ala Pro Cys Val Phe Gly Gly Ser Val Tyr Arg Ser Gly Glu
   100 105 110
   Ser Phe Gln Ser Ser Cys Lys Tyr Gln Cys Thr Cys Leu Asp Gly Ala
   115 120 125
   Val Gly Cys Val Pro Leu Cys Ser Met Asp Val Arg Leu Pro Ser Pro
   130 135 140
   Asp Cys Pro Phe Pro Arg Arg Val Lys Leu Pro Gly Lys Cys Cys Glu
   145 150 155 160
   Glu Trp Val Cys Asp Glu Pro Lys Asp Arg Thr Val Val Gly Pro Ala
   165 170 175
   Leu Ala Ala Tyr Arg Leu Glu Asp Thr Phe Gly Pro Asp Pro Thr Met
   180 185 190
   Met Arg Ala Asn Cys Leu Val Gln Thr Thr Glu Trp Ser Ala Cys Ser
   195 200 205
   Lys Thr Cys Gly Met Gly Ile Ser Thr Arg Val Thr Asn Asp Asn Thr
   210 215 220
   Phe Cys Arg Leu Glu Lys Gln Ser Arg Leu Cys Met Val Arg Pro Cys
   225 230 235 240
   Glu Ala Asp Leu Glu Glu Asn Ile Lys Lys Gly Lys Lys Cys Ile Arg
   245 250 255
   Thr Pro Lys Ile Ala Lys Pro Val Lys Phe Glu Leu Ser Gly Cys Thr
   260 265 270
   Ser Val Lys Thr Tyr Arg Ala Lys Phe Cys Gly Val Cys Thr Asp Gly
   275 280 285
   Arg Cys Cys Thr Pro His Arg Thr Thr Thr Leu Pro Val Glu Phe Lys
   290 295 300
   Cys Pro Asp Gly Glu Ile Met Lys Lys Asn Met Met Phe Ile Lys Thr
   305 310 315 320
   Cys Ala Cys His Tyr Asn Cys Pro Gly Asp Asn Asp Ile Phe Glu Ser
   325 330 335
   Leu Tyr Tyr Arg Lys Met Tyr Gly Asp Met Ala
   340 345
<210> 5
   <211> 18
   <212> DNA
   <213> Artificial
   <220>
   <223> primer1
   <400> 5
   gagcccaagg accaaacc 18
<210> 6
   <211> 18
   <212> DNA
   <213> Artificial
   <220>
   <223> primer2
   <400> 6
   ggccaaacgt gtcttcca 18
<210> 7
   <211> 21
   <212> DNA
   <213> Artificial
   <220>
   <223> probe1
   <400> 7
   ctgccctcgc ggcttaccga c 21
<210> 8
   <211> 19
   <212> DNA
   <213> Artificial
   <220>
   <223> primer3
   <400> 8
   gtgtgtgatg agcccaagg 19
<210> 9
   <211> 20
   <212> DNA
   <213> Artificial
   <220>
   <223> primer4
   <400> 9
   gtcagggcca aatgtgtctt 20
<210> 10
   <211> 24
   <212> DNA
   <213> Artificial
   <220>
   <223> probe2
   <400> 10
   ctgccctagc tgcctaccga ctgg 24